# EUROPEAN PATENT SPECIFICATION

(11) **EP 1 043 995 B1**
(45) Date of publication and mention of the grant of the patent: **29.11.2006**
(21) Application number: 98964708.6
(22) Date of filing: 22.12.1998
(51) Int. Cl.: A61K 31/38, A61K 31/415, A61K 31/44

(54) **INHIBITION OF p38 KINASE ACTIVITY USING ARYL AND HETEROARYL SUBSTITUTED HETEROCYCLIC UREAS**
INHIBIERUNG DER p38 KINASE-AKTIVITÄT DURCH DIE VERWENDUNG VON ARYL- UND HETEROARYLSUBSTITUIERTEN HARNSTOFFEN
INHIBITION DE L'ACTIVITE DE p38 KINASE AU MOYEN D'UREES HETEROCYCLIQUES ARYLE ET HETEROARYLE SUBSTITUEES

(30) Priority: 22.12.1997 US 995751
(43) Date of publication of application: 18.10.2000
(73) Proprietor: Bayer Pharmaceuticals Corp., West Haven, CT 06516 (US)
(72) Inventor: DUMAS, Jacques, Orange, CT 06477 (US); KHIRE, Uday, Hamden, CT 06518 (US); LOWINGER, Timothy, Bruno, Hyogo 662-0046 (JP); RIEDL, Bernd, Branford, CT 06405 (US); SCOTT, William, J., Guilford, CT 06437 (US); SMITH, Roger, A., Madison, CT 06443 (US); WOOD, Jill, E., Hamden, CT 06517 (US); HATOUM-MOKDAD, Holia, Hamden, CT 06514 (US); JOHNSON, Jeffrey, Branford, CT 06405-6122 (US); REDMAN, Aniko, Derby, CT 06418 (US); SIBLEY, Robert, North Haven, CT 06473 (US)
(74) Representative: Weiss, Wolfgang
(86) International application number: PCT/US1998/026079
(87) International publication number: WO 1999/032110

(56) References cited:
- WO-A-95/07922
- WO-A-96/40673
- WO-A-99/23091
- WO-A-99/32455
- US-A- 5 162 360
- US-A- 5 319 099
- US-A- 5 559 137
- WILSON ET AL: "The structural basis for the specificity of pyridinylimidazole inhibitors of p38 MAP kinase" CHEMISTRY AND BIOLOGY, CURRENT BIOLOGY, LONDON, GB, vol. 4, no. 4, June 1997 (1997-06), pages 423-431, XP002103155 ISSN: 1074-5521
- HANSON G J: "Inhibitors of p38 kinase" EXPERT OPINION ON THERAPEUTIC PATENTS, ASHLEY PUBLICATIONS, GB, vol. 7, no. 7, 1997, pages 729-733, XP002086152 ISSN: 1354-3776

## Description

### Field of the Invention

This invention relates to the use of a group of aryl ureas in treating cytokine mediated diseases and proteolytic enzyme mediated diseases, and pharmaceutical compositions for use in such therapy.

### Background of the Invention

Two classes of effector molecules which are critical for the progression of rheumatoid arthritis are pro-inflammatory cytokines and tissue degrading proteases. Recently, a family of kinases was described which is instrumental in controlling the transcription and translation of the structural genes coding for these effector molecules.

The mitogen-activated protein (MAP) kinase family is made up of a series of structurally related proline-directed serine/threonine kinases which are activated either by growth factors (such as EGF) and phorbol esters (ERK), or by IL-1, TNFα or stress (p38, JNK). The MAP kinases are responsible for the activation of a wide variety of transcription factors and proteins involved in transcriptional control of cytokine production. A pair of novel protein kinases involved in the regulation of cytokine synthesis was recently described by a group from SmithKline Beecham (Lee et al. *Nature* **1994,** *372*, 739). These enzymes were isolated based on their affinity to bond to a class of compounds, named CSAIDSs (cytokine suppressive antiinflammatory drugs) by SKB. The CSAIDs, pyridinyl imidazoles, have been shown to have cytokine inhibitory activity both *in vitro* and *in vivo.* The isolated enzymes, CSBP-1 and -2 (CSAID binding protein 1 and 2) have been cloned and expressed. A murine homologue for CSBP-2, p38, has also been reported (Han et al. *Science* **1994**, *265*, 808).

Early studies suggested that CSAIDs function by interfering with m-RNA translational events during cytokine biosynthesis. Inhibition of p38 has been shown to inhibit both cytokine production (eg., TNFα, IL-1, IL-6, IL-8) and proteolytic enzyme production (eg., MMP-1, MMP-3) *in vitro* and/or *in vivo.*

Clinical studies have linked TNFα production and/or signaling to a number of diseases including rheumatoid arthritis (Maini. *J. Royal Coll. Physicians London* **1996,** *30,* 344). In addition, excessive levels of TNFα have been implicated in a wide variety of inflammatory and/or immunomodulatory diseases, including acute rheumatic fever (Yegin et al. *Lancet* **1997**, *349,* 170), bone resorption (Pacifici et al. *J. Clin. Endocrinol. Metabol.* **1997,** *82,* 29), postmenopausal osteoperosis (Pacifici et al. *J. Bone Mineral Res.* **1996,** *11,* 1043), sepsis (Blackwell et al. *Br. J. Anaesth.* **1996,** *77*, 110), gram negative sepsis (Debets et al. *Prog. Clin. Biol. Res.* **1989,** *308,* 463), septic shock (Tracey et al. *Nature* **1987***, 330,* 662; Girardin et al. *New England J. Med.* **1988,** *319,* 397), endotoxic shock (Beutler et al. *Science* **1985,** *229,* 869; Ashkenasi et al. *Proc. Nat'l. Acad. Sci. USA* **1991,** *88,* 10535), toxic shock syndrome, (Saha et al. *J. Immunol.* **1996,** *157,* 3869; Lina et al. *FEMS Immunol. Med. Microbiol.* **1996,** *13,* 81), systemic inflammatory response syndrome (Anon. *Crit. Care Med.* **1992,** *20,* 864), inflammatory bowel diseases (Stokkers et al. *J. Inflamm.* **1995-6,** *47,* 97) including Crohn's disease (van Deventer et al. *Aliment. Pharmacol. Therapeu.* **1996,** *10 (Suppl. 2),* 107; van Dullemen et al. *Gastroenterology* **1995,** *109,* 129) and ulcerative colitis (Masuda et al. *J. Clin. Lab. Immunol.* **1995,** *46*, 111), Jarisch-Herxheimer reactions (Fekade et al. *New England J. Med.* **1996,** *335,* 311), asthma (Amrani et al. *Rev. Malad. Respir.* **1996**, *13*, 539), adult respiratory distress syndrome (Roten et al. *Am. Rev. Respir. Dis.* **1991**, *143,* 590*;* Suter et al. *Am. Rev. Respir. Dis.* **1992,** *145,* 1016), acute pulmonary fibrotic diseases (Pan et al. *Pathol. Int.* **1996,** *46,* 91), pulmonary sarcoidosis (Ishioka et al. *Sarcoidosis Vasculitis Diffuse Lung Dis.* **1996,** *13,* 139), allergic respiratory diseases (Casale et al. *Am. J. Respir. Cell Mol. Biol.* **1996,** *15,* 35), silicosis (Gossart et al. *J. Immunol.* **1996,** *156,* 1540; Vanhee et al. *Eur. Respir. J.* **1995,** *8,* 834), coal worker's pneumoconiosis (Borm et al. *Am. Rev. Respir. Dis.* **1988,** *138,* 1589), alveolar injury (Horinouchi et al. *Am. J. Respir. Cell Mol. Biol.* **1996,** *14,* 1044), hepatic failure (Gantner et al. *J. Pharmacol. Exp. Therap.* **1997,** *280,* 53), liver disease during acute inflammation (Kim et al. *J. Biol. Chem.* **1997,** *272,* 1402), severe alcoholic hepatitis (Bird et al. *Ann. Intern. Med.* **1990,** *112,* 917), malaria (Grau et al. *Immunol. Rev.* **1989,** *112,* 49; Taverne et al. *Parasitol. Today* **1996,** *12*, 290) including Plasmodium falciparum malaria (Perlmann et al. *Infect. Immunit.* **1997**, *65,* 116) and cerebral malaria (Rudin et al. *Am. J. Pathol.* **1997,** *150,* 257), non-insulin-dependent diabetes mellitus (NIDDM; Stephens et al. *J. Biol. Chem.* **1997**, *272,* 971; Ofei et al. *Diabetes* **1996**, *45,* 881), congestive heart failure (Doyama et al. *Int. J. Cardiol.* **1996**, *54*, 217; McMurray et al. *Br. Heart J.* **1991**, *66*, 356), damage following heart disease (Malkiel et al. *Mol. Med. Today* **1996**, *2*, 336), atherosclerosis (Parums et al. *J. Pathol.* **1996**, *179,* A46), Alzheimer's disease (Fagarasan et al. *Brain Res.* **1996**, *723,* 231*;* Aisen et al. *Gerontology* **1997**, *43*, 143), acute encephalitis (Ichiyama et al. *J. Neurol.* **1996,** *243,* 457), brain injury (Cannon et al. *Crit. Care Med.* **1992***, 20,* 1414; Hansbrough et al. *Surg. Clin. N. Am.* **1987,** *67,* 69; Marano et al. *Surg. Gynecol. Obstetr.* **1990,** *170,* 32), multiple sclerosis (M.S.; Coyle. *Adv. Neuroimmunol.* **1996,** *6,* 143; Matusevicius et al. *J. Neuroimmunol.* **1996,** *66,* 115) including demyelation and oligiodendrocyte loss in multiple sclerosis (Brosnan et al. *Brain Pathol.* **1996,** *6*, 243), advanced cancer (MucWierzgon et al. *J. Biol. Regulators Homeostatic Agents* **1996,** *10,* 25), lymphoid malignancies (Levy et al. *Crit. Rev. Immunol.* **1996,** *16,* 31), pancreatitis (Exley et al. *Gut* **1992**, *33*, 1126) including systemic complications in acute pancreatitis (McKay et al. *Br. J. Surg.* **1996,** *83*, 919), impaired wound healing in infection inflammation and cancer (Buck et al. *Am. J. Pathol.* **1996,** *149,* 195), myelodysplastic syndromes (Raza et al. *Int. J. Hematol.* **1996,** *63,* 265), systemic lupus erythematosus (Maury et al. *Arthritis Rheum.* **1989,** *32,* 146), biliary cirrhosis (Miller et al. *Am. J. Gasteroenterolog.* **1992,** *87,* 465), bowel necrosis (Sun et al. *J. Clin. Invest.* **1988,** *81,* 1328), psoriasis (Christophers. *Austr. J. Dermatol.* **1996**, *37*, S4), radiation injury (Redlich et al. *J. Immunol.* **1996**, *157,* 1705), and toxicity following administration of monoclonal antibodies such as OKT3 (Brod et al. *Neurology* **1996**, *46,* 1633). TNFα levels have also been related to host-versus-graft reactions (Piguet et al. *Immunol. Ser.* **1992**, *56,* 409) including ischemia reperfusion injury (Colletti et al. *J. Clin. Invest.* **1989**, *85*, 1333) and allograft rejections including those of the kidney (Maury et al. *J. Exp. Med.* **1987**, *166*, 1132), liver (Imagawa et al. *Transplantation* **1990**, *50,* 219), heart (Bolling et al. *Transplantation* **1992**, *53*, 283), and skin (Stevens et al. *Transplant. Proc.* **1990**, *22*, 1924), lung allograft rejection (Grossman et al. *Immunol. Allergy Clin. N. Am.* **1989**, *9*, 153) including chronic lung allograft rejection (obliterative bronchitis; LoCicero et al. *J. Thorac. Cardiovasc. Surg.* **1990**, *99*, 1059), as well as complications due to total hip replacement (Cirino et al. *Life Sci.* **1996**, *59,* 86). TNFα has also been linked to infectious diseases (review: Beutler et al. *Crit. Care Med.* **1993**, *21*, 5423; Degre. *Biotherapy* **1996**, *8*, 219) including tuberculosis (Rook et al. *Med Malad. Infect.* **1996,** *26*, 904), Helicobacter pylori infection during peptic ulcer disease (Beales et al. *Gastroenterology* **1997,** *112*, 136), Chaga's disease resulting from Trypanosoma cruzi infection (Chandrasekar et al. *Biochem. Biophys. Res. Commun.* **1996**, *223,* 365), effects of Shiga-like toxin resulting from E. coli infection (Harel et al. *J. Clin. Invest.* **1992,** *56,* 40), the effects of enterotoxin A resulting from Staphylococcus infection (Fischer et al. *J. Immunol.* **1990,** *144,* 4663), meningococcal infection (Waage et al. *Lancet* **1987,** 355; Ossege et al. *J. Neurolog. Sci.* **1996,** *144,* 1), and infections from Borrelia burgdorferi (Brandt et al. *Infect. Immunol.* **1990,** *58,* 983), Treponema pallidum (Chamberlin et al. *Infect. Immunol.* **1989,** *57*, 2872), cytomegalovirus (CMV; Geist et al. *Am. J. Respir. Cell Mol. Biol.* **1997,** *16,* 31), influenza virus (Beutler et al. *Clin. Res.* **1986,** *34,* 491a), Sendai virus (Goldfield et al. *Proc. Nat'l. Acad. Sci. USA* **1989,** *87,* 1490), Theiler's encephalomyelitis virus (Sierra et al. *Immunology* **1993**, *78,* 399), and the human immunodeficiency virus (HIV; Poli. *Proc. Nat'l. Acad. Sci. USA* **1990**, *87*, 782; Vyakaram et al. *AIDS* **1990,** *4,* 21; Badley et al. *J. Exp. Med.* **1997**,*185*, 55).

Because inhibition of p38 leads to inhibition of TNFα production, p38 inhibitors will be useful in treatment of the above listed diseases.

A number of diseases are thought to be mediated by excess or undesired matrix-destroying metalloprotease (MMP) activity or by an imbalance in the ratio of the MMPs to the tissue inhibitors of metalloproteinases (TIMPs). These include osteoarthritis (Woessner et al. *J. Biol. Chem.* **1984**, *259*, 3633), rheumatoid arthritis (Mullins et al. *Biochim. Biophys. Acta* **1983**, *695,* 117; Woolley et al. *Arthritis Rheum.* **1977**, *20*, 1231; Gravallese et al. *Arthritis Rheum.* **1991**, *34*, 1076), septic arthritis (Williams et al. *Arthritis Rheum.* **1990,** *33,* 533), tumor metastasis (Reich et al. *Cancer Res.* **1988,** *48,* 3307; Matrisian et al. *Proc. Nat'l. Acad. Sci., USA* **1986,** *83,* 9413), periodontal diseases (Overall et al. *J. Periodontal Res.* **1987,** *22,* 81), corneal ulceration (Bums et al. *Invest. Opthalmol. Vis. Sci.* **1989,** *30,* 1569), proteinuria (Baricos et al. *Biochem. J.* **1988***, 254,* 609), coronary thrombosis from atherosclerotic plaque rupture (Henney et al. *Proc. Nat'l. Acad. Sci., USA* **1991**, *88*, 8154), aneurysmal aortic disease (Vine et al. *Clin. Sci.* **1991**, *81*, 233), birth control (Woessner et al. *Steroids* **1989**, *54*, 491), dystrophobic epidermolysis bullosa (Kronberger et al. *J. Invest. Dermatol.* **1982**, *79*, 208), degenerative cartilage loss following traumatic joint injury, osteopenias mediated by MMP activity, tempero mandibular joint disease, and demyelating diseases of the nervous system (Chantry et al. *J. Neurochem.* **1988,** *50,* 688).

Because inhibition of p38 leads to inhibition of MMP production, p38 inhibitors will be useful in treatment of the above listed diseases.

Inhibitors of p38 are active in animal models of TNFα production, including a muirne lipopolysaccharide (LPS) model of TNFα production. Inhibitors of p38 are active in a number of standard animal models of inflammatory diseases, including carrageenan-induced edema in the rat paw, arachadonic acid-induced edema in the rat paw, arachadonic acid-induced peritonitis in the mouse, fetal rat long bone resorption, murine type II collagen-induced arthritis, and Fruend's adjuvant-induced arthritis in the rat. Thus, inhibitors of p38 will be useful in treating diseases mediated by one or more of the above-mentioned cytokines and/or proteolytic enzymes.

The need for new therapies is especially important in the case of arthritic diseases. The primary disabling effect of osteoarthritis, rheumatoid arthritis and septic arthritis is the progressive loss of articular cartilage and thereby normal joint function. No marketed pharmaceutical agent is able to prevent or slow this cartilage loss, although nonsteroidal antiinflammatory drugs (NSAIDs) have been given to control pain and swelling. The end result of these diseases is total loss of joint function which is only treatable by joint replacement surgery. P38 inhibitors will halt or reverse the progression of cartilage loss and obviate or delay surgical intervention.

Several patents have appeared claiming polyarylimidazoles and/or compounds containing polyarylimidazoles as inhibitors of p38 (for example, Lee et al. WO 95/07922; Adams et al. WO 95/02591; Adams et al. WO 95/13067; Adams et al. WO 95/31451). It has been reported that arylimidazoles complex to the ferric form of cytochrome P450_{cam} (Harris et al. *Mol. Eng.* **1995,** *5,* 143, and references therein), causing concern that these compounds may display structure-related toxicity (Howard-Martin et al. *Toxicol. Pathol.* **1987**, *15,* 369). Therefore, there remains a need for improved p38 inhibitors.

US 5,162,360 relates to N-substituted aryl-N'-heterocyclic substituented ureas for the treatment of hypercholesterolemia and atherosclerosis.

WO 96/40673 relates to the use of phenyl ureas and thioureas to prevent and treat cell proliferative disorders or cell differentiation disorders associated with particular tyrosine kinases by inhibiting abnormal tyrosine kinase activities.

### Summary of the Invention

This invention provides compounds, generally described as aryl ureas, including both aryl and heteroaryl analogues, which inhibit p38 mediated events and thus inhibit the production of cytokines (such as TNFα, IL-1 and IL-8) and proteolytic enzymes (such as MMP-1 and MMP-3). The invention also provides the use of the compounds of the present invention for the preparation of a medicament for treating a cytokine mediated disease state in humans or mammals, wherein the cytokine is one whose production is affected by p38. Examples of such cytokines include, but are not limited to TNFα, IL-1 and IL-8. The invention also provides the use of the compounds of the present invention for the preparation of a medicament for treating a protease mediated disease state in humans or mammals, wherein the protease is one whose production is affected by p38. Examples of such proteases include, but are not limited to collagenase (MMP-1) and stromelysin (MMP-3).

Accordingly, these compounds are useful therapeutic agents for such acute and chronic inflammatory and/or immunomodulatory diseases other than cancer, such as rheumatoid arthritis, osteoarthritis, septic arthritis, rheumatic fever, bone resorption, osteoporosis, postmenopausal osteoperosis, sepsis, gram negative sepsis, septic shock, endotoxic shock, toxic shock syndrome, systemic inflammatory response syndrome, inflammatory bowel diseases including Crohn's disease and ulcerative colitis, Jarisch-Herxheimer reactions, asthma, adult respiratory distress syndrome, acute pulmonary fibrotic diseases, pulmonary sarcoidosis, allergic respiratory diseases, silicosis, coal worker's pneumoconiosis, alveolar injury, hepatic failure, liver disease during acute inflammation, severe alcoholic hepatitis, malaria including Plasmodium falciparum malaria and cerebral malaria, non-insulin-dependent diabetes mellitus (NIDDM), congestive heart failure, damage following heart disease, atherosclerosis, Alzheimer's disease, acute encephalitis, brain injury, multiple sclerosis including demyelation and oligiodendrocyte loss in multiple sclerosis, lymphoid malignancies, pancreatitis, including systemic complications in acute pancreatitis, impaired wound healing in infection, inflammation, periodontal diseases, corneal ulceration, proteinuria, myelodysplastic syndromes, systemic lupus erythematosus, biliary cirrhosis, bowel necrosis, psoriasis, radiation injury, toxicity following administration of monoclonal antibodies such as OKT3, host-versus-graft reactions including ischemia reperfusion injury and allograft rejections including kidney, liver, heart, and skin allograft rejections, lung allograft rejection including chronic lung allograft rejection (obliterative bronchitis) as well as complications due to total hip replacement, and infectious diseases including tuberculosis, Helicobacter pylori infection during peptic ulcer disease, Chaga's disease resulting from Trypanosoma cruzi infection, effects of Shiga-like toxin resulting from E. coli infection, effects of enterotoxin A resulting from Staphylococcus infection, meningococcal infection, and infections from Borrelia burgdorferi, Treponema pallidum, cytomegalovirus, influenza virus, Theiler's encephalomyelitis virus, and the human immunodeficiency virus (HIV).

The present invention, therefore, provides compounds generally described as aryl ureas, including both aryl and heteroaryl analogues, which inhibit the p38 pathway. The invention also provides the use of the compounds of the present invention for the preparation of a medicament for treatment of p38-mediated disease states in humans or mammals, e.g., disease states mediated by one or more cytokines or proteolytic enzymes produced and/or activated by a p38 mediated process. Thus, the invention is directed to compounds of Formula I and its use for the preparation of a medicament for the treatment of diseases mediated by p38 kinase, 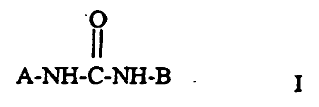 wherein B is generally an up to tricyclic, aryl or heteroaryl moiety with up to 30 carbon atoms with at least one 5 or 6 member aromatic structure containing 0-4 members of the group consisting of nitrogen, oxygen and sulfur substituted by -Y-Ar, and B is optionally substituted by one or more substituents, independently selected from the group consisting of halogen, up to per-halosubstitution, and Xₙ, therein n is 0-2. A is a heteroaryl moiety discussed in more detail below.

The aryl and heteroaryl moiety of B may contain separate cyclic structures and can include a combination of aryl, heteroaryl and cycloalkyl structures. The substituents for these aryl and heteroaryl moieties can vary widely and include halogen, hydrogen, hydrosulfide, cyano, nitro, amines and various carbon-based moieties, including those which contain one or more of sulfur, nitrogen, oxygen and/or halogen and are discussed more particularly below.

Suitable aryl and heteroaryl moieties for B of formula I include, but are not limited to aromatic ring structures containing 4-30 carbon atoms and 1-3 rings, at least one of which is a 5-6 member aromatic ring. One or more of these rings may have 1-4 carbon atoms replaced by oxygen, nitrogen and/or sulfur atoms.

Examples of suitable aromatic ring structures include phenyl, pyridinyl, naphthyl, pyrimidinyl, benzothiozolyl, quinoline, isoquinoline, phthalimidinyl and combinations thereof, such as diphenyl ether (phenyloxyphenyl), diphenyl thioether (phenylthiophenyl), phenylaminophenyl, phenylpyridinyl ether (pyridinyloxyphenyl), pyridinylmethylphenyl, phenylpyridinyl thioether (pyridinylthiophenyl), phenylbenzothiazolyl ether (benzothiazolyloxyphenyl), phenylbenzothiazolyl thioether (benzothiazolylthiophenyl), phenylpyrimidinyl ether, phenylquinoline thioether, phenylnaphthyl ether, pyridinylnapthyl ether, pyridinylnaphthyl thioether, and phthalimidylmethylphenyl.

Examples of suitable heteroaryl groups include, but are not limited to, 5-12 carbon-atom aromatic rings or ring systems containing 1-3 rings, at least one of which is aromatic, in which one or more, e.g., 1-4 carbon atoms in one or more of the rings can be replaced by oxygen, nitrogen or sulfur atoms. Each ring typically has 3-7 atoms. For example, B can be 2- or 3-furyl, 2- or 3-thienyl, 2- or 4-triazinyl, 1-, 2- or 3-pyrrolyl, 1-, 2-, 4- or 5-imidazolyl, 1-, 3-, 4- or 5-pyrazolyl, 2-, 4- or 5-oxazolyl, 3-, 4-or 5-isoxazolyl, 2-, 4- or 5-thiazolyl, 3-, 4- or 5-isothiazolyl, 2-, 3- or 4-pyridyl, 2-, 4-, 5- or 6-pyrimidinyl, 1,2,3-triazol-1-, -4- or-5-yl, 1,2,4-triazol-1-, -3- or-5-yl, 1- or 5-tetrazolyl, 1,2,3-oxadiazol-4- or -5-yl, 1,2,4-oxadiazol-3- or -5-yl, 1,3,4-thiadiazol-2-or -5-yl, 1,2,4-oxadiazol-3- or -5-yl, 1,3,4-thiadiazol-2- or -5-yl, 1,3,4-thiadiazol-3-or -5-yl, 1,2,3-thiadiazol-4- or -5-yl, 2-, 3-, 4-, 5- or 6-2H-thiopyranyl, 2-, 3- or 4-4H-thiopyranyl, 3- or 4-pyridazinyl, pyrazinyl, 2-, 3-, 4-, 5-, 6- or 7-benzofuryl, 2-, 3-, 4-, 5-, 6- or 7-benzothienyl, 1-, 2-, 3-, 4-, 5-, 6- or 7-indolyl, 1-, 2-, 4- or 5-benzimidazolyl, 1-, 3-, 4-, 5-, 6- or 7-benzopyrazolyl, 2-, 4-, 5-, 6- or 7-benzoxazolyl, 3-, 4-, 5- 6- or 7-benzisoxazolyl, 1-, 3-, 4-, 5-, 6- or 7-benzothiazolyl, 2-, 4-, 5-, 6- or 7-benzisothiazolyl, 2-, 4-, 5-, 6- or 7-benz-1,3-oxadiazolyl, 2-, 3-, 4-, 5-, 6-, 7- or 8-quinolinyl, 1-, 3-, 4-, 5-, 6-, 7-, 8- isoquinolinyl, 1-, 2-, 3-, 4- or 9-carbazolyl, 1-, 2-, 3-, 4-, 5-, 6-, 7-, 8- or 9-acridinyl, or 2-, 4-, 5-, 6-, 7- or 8-quinazolinyl, or additionally optionally substituted phenyl, 2- or 3-thienyl, 1,3,4-thiadiazolyl, 3-pyrryl, 3-pyrazolyl, 2-thiazolyl or 5-thiazolyl, etc. For example, B can be 4-methyl-phenyl, 5-methyl-2-thienyl, 4-methyl-2-thienyl, 1-methyl-3-pyrryl, 1-methyl-3-pyrazolyl, 5-methyl-2-thiazolyl or 5-methyl-1,2,4-thiadiazol-2-yl.

Suitable alkyl groups and alkyl portions of groups, e.g., alkoxy, etc., throughout include methyl, ethyl, propyl, butyl, etc., including all straight-chain and branched isomers such as isopropyl, isobutyl, *sec*-butyl, *tert*-butyl, etc.

Suitable aryl groups include, for example, phenyl and 1- and 2-naphthyl.

Suitable cycloalkyl groups include cyclopropyl, cyclobutyl, cyclohexyl, etc. The term "cycloalkyl", as used herein, refers to cyclic structures with or without alkyl substituents such that, for example, "C₄ cycloalkyl" includes methyl substituted cyclopropyl groups as well as cyclobutyl groups. The term "cycloalkyl also includes saturated heterocycles.

Suitable halogens include F, Cl, Br, and/or I, from one to persubstitution (i.e., all H atoms on the group are replaced by halogen atom), being possible, mixed substitution of halogen atom types also being possible on a given moiety.

As indicated above, these ring systems can be unsubstituted or substituted by substituents such as halogen up to per-halosubstitution. Other suitable substituents for the moieties of B are generally referred to as X and X' herein, and are independently selected from the group consisting of -CN, -CO₂R⁵, -C(O)NR⁵R^{5'}, -C(O)R⁵, -NO₂, -OR⁵, -SR⁵, -NR⁵R^{5'}, -NR⁵C(O)OR^{5'}, -NR⁵C(O)R^{5'}, C₁-C₁₀ alkyl, C₂₋₁₀-alkenyl, C₁₋₁₀-alkoxy, C₃-C₁₀ cycloalkyl, C₆-C₁₄ aryl, C₇-C₂₄ alkaryl, C₃-C₁₃ heteroaryl, C₄-C₂₃ alkheteroaryl, substituted C₁-C₁₀ alkyl, substituted C₂₋₁₀-alkenyl, substituted C₁₋₁₀-alkoxy, substituted C₃-C₁₀ cycloalkyl, substituted C₄-C₂₃ alkheteroaryl and -Y-Ar.

Where a substituent, X or X', is a substituted group, it is substituted by one or more substituents independently selected from the group consisting of -CN, -CO₂R⁵, -C(O)R⁵, -C(O)NR⁵R^{5'}, -OR⁵, -SR⁵, -NR⁵R^{5'}, -NO₂, -NR⁵C(O)R^{5'}, -NR⁵C(O)OR^{5'} and halogen up to per-halo substitution.

The moieties R⁵ and R^{5'} are independently selected from H, C₁-C₁₀ alkyl, C₂₋₁₀-alkenyl, C₃-C₁₀ cycloalkyl, C₆-C₁₄ aryl, C₃-C₁₃ heteroaryl, C₇-C₂₄ alkaryl, C₄-C₂₃ alkheteroaryl, up to per-halosubstituted C₁-C₁₀ alkyl, up to per-halosubstituted C₂₋₁₀-alkenyl , up to per-halosubstituted C₃-C₁₀ cycloalkyl, up to per-halosubstituted C₆-C₁₄ aryl and up to per-halosubstituted C₃-C₁₃ heteroaryl.

The bridging group Y is selected from the group consisting of -O-, -S-, -N(R⁵)-, -(CH₂)-ₘ, -C(O)-, -CH(OH)-, -NR⁵C(O)NR⁵R^{5'}-, -NR⁵C(O)-, -C(O)NR⁵-, -(CH₂)ₘO-, -(CH₂)ₘS-, -(CH₂)ₘN(R⁵)-, -O(CH₂)ₘ-, -CHX^{a}, -CX^{a}₂-, -S-(CH₂)ₘ- and -N(R-⁵)(CH₂)ₘ-, where m = 1-3, and X^{a} is halogen.

The moiety Ar is a 5-10 member aromatic structure containing 0-4 members of the group consisting of nitrogen, oxygen and sulfur which is unsubstituted or substituted by halogen up to per-halosubstitution and optionally substituted by Zₙ₁, wherein n1 is 0 to 3.

Each Z substituent is independently selected from the group consisting of -CN, -CO₂R⁵, -C(O)NR⁵R^{5'}, -C(O)-NR⁵H, -NO₂, -OR⁵, -SR⁵, -NR⁵R^{5'}, -NR⁵C(O)OR^{5'}, -C(O)R⁵, -NR⁵C(O)R^{5'}, C₁-C₁₀ alkyl, C₃-C₁₀ cycloalkyl, C₆-C₁₄ aryl, C₃-C₁₃ heteroaryl, C₇-C₂₄ alkaryl, C₄-C₂₃ alkheteroaryl, substituted C₁-C₁₀ alkyl, substituted C₃-C₁₀ cycloalkyl, substituted C₇-C₂₄ alkaryl and substituted C₄-C₂₃ alkheteroaryl. If Z is a substituted group, it is substituted by the one or more substituents independently selected from the group consisting of -CN, -CO₂R⁵, -C(O)NR⁵R^{5'}, - OR⁵, -SR⁵, -NO₂, -NR⁵R^{5'}, -NR⁵C(O)R^{5'} and -NR⁵C(O)OR^{5'}.

The aryl and heteroaryl moieties of B of Formula I are preferably selected from the group consisting of 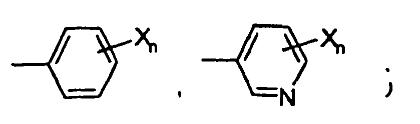 which are unsubstituted or substituted by halogen, up to per-halosubstitution. X is as defined above and n = 1-3 and at least one X is -Y-Ar.

The aryl and heteroaryl moieties of B are more preferably of the formula: 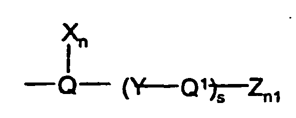 wherein Y is selected from the group consisting of -O-, -S-, -CH₂-, -SCH₂-, -CH₂S-, -CH(OH)-, -C(O)-, -CX^{a}₂, -CX^{a}H-, -CH₂O- and -OCH₂- and X^{a} is halogen.

Q is a six member aromatic structure containing 0-2 nitrogen, substituted or unsubstituted by halogen, up to per-halosubstitution and Q¹ is a mono- or bicyclic aromatic structure of 3 to 10 carbon atoms and 0-4 members of the group consisting of N, O and S, unsubstituted or unsubstituted by halogen up to per-halosubstitution. X, Z, n and n 1 are as defined above and s = 0 or 1.

In preferred embodiments, Q is phenyl or pyridinyl, substituted or unsubstituted by halogen, up to per-halosubstitution and Q¹ is selected from the group consisting of phenyl, pyridinyl, naphthyl, pyrimidinyl, quinoline, isoquinoline, imidazole and benzothiazolyl, substituted or unsubstituted by halogen, up to per-halo substitution, or -Y-Q¹ is phthalimidinyl substituted or unsubstituted by halogen up to per-halo substitution. Z and X are preferably independently selected from the group consisting of -R⁶, -OR⁶ and -NHR⁷, wherein R⁶ is hydrogen, C₁-C₁₀-alkyl or C₃-C₁₀-cycloalkyl and R⁷ is preferably selected from the group consisting of hydrogen, C₃-C₁₀-alkyl, C₃-C₆-cycloalkyl and C₆-C₁₀-aryl, wherein R⁶ and R⁷ can be substituted by halogen or up to per-halosubstitution.

The heteroaryl moiety A of formula I is selected from the group consisting of: 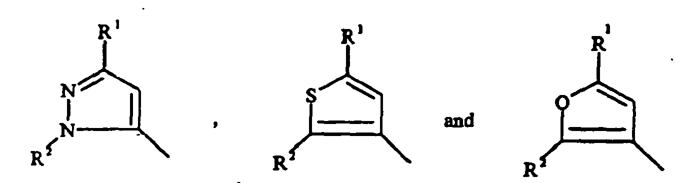 wherein R¹ is selected from the group consisting of C₃-C₁₀ alkyl, C₃-C₁₀ cycloalkyl, up to per-halosubstituted C₁-C₁₀ alkyl and up to per-halosubstituted C₃-C₁₀ cycloalkyl and R² is C₆-C₁₄ aryl, C₃-C₁₄ heteroaryl, substituted C₆-C₁₄ aryl or substituted C₃-C₁₄ heteroaryl.

Where R² is a substituted group, it is substituted by one or more substituents independently selected from the group consisting of halogen, up to per-halosubstitution, and Vₙ, where n = 0-3.

Each V is independently selected from the group consisting of -CN, -OC(O)NR⁵R^{5'}, -CO₂R⁵, -C(O)NR⁵R^{5'}, -OR⁵, -SR⁵, -NR⁵R^{5'}, -C(O)R⁵, -NR⁵C(O)OR^{5'}, -SO₂R⁵, -SOR⁵, -NR⁵C(O)R^{5'}, -NO₂, C₁-C₁₀ alkyl, C₃-C₁₀ cycloalkyl, C₆-C₁₄ aryl, C₃-C₁₃ heteroaryl, C₇-C₂₄ alkaryl, C₄-C₂₄ alkheteroaryl, substituted C₁-C₁₀ alkyl, substituted C₃-C₁₀ cycloalkyl, substituted C₆-C₁₄ aryl, substituted C₃-C₁₃ heteroaryl, substituted C₇-C₂₄ alkaryl and substituted C₄-C₂₄ alkheteroaryl.

If V is a substituted group, it is substituted by one or more substituents independently selected from the group consisting of halogen, up to per-halosubstitution, -CN, -CO₂R⁵, -C(O)R⁵, -C(O)NR⁵R⁵, - NR⁵R^{5'}, -OR⁵, -SR⁵, - NR⁵C(O)R^{5'}, -NR⁵C(O)OR^{5'} and -NO₂.

The compound of the present invention is preferably one of the formulae 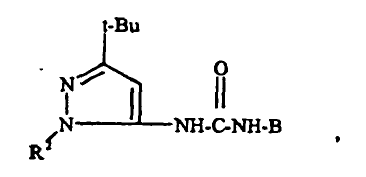 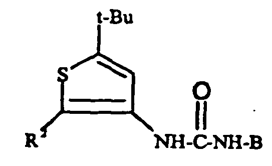 or 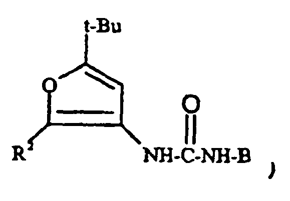 or a pharmaceutically acceptable salt thereof,
wherein B and R² are as defined herein.

The substituents R⁵ and R^{5'} are preferably each independently selected form the group consisting of H, C₁-C₁₀ alkyl, C₃-C₁₀ cycloalkyl, C₆-C₁₄ aryl, C₃-C₁₃ heteroaryl, C₇-C₂₄ alkaryl, C₄-C₂₃ alkheteroaryl, up to per-halosubstituted C₁-C₁₀ alkyl, up to per-halosubstituted C₃-C₁₀ cycloalkyl, up to per-halosubstituted C₆-C₁₄ aryl and up to per-halosubstituted C₃-C₁₃ heteroaryl.

R² is preferably substituted or unsubstituted phenyl or pyridinyl, where the substituents for R² are selected from the group consisting of halogen, up to per-halosubstituition and Vₙ¹, wherein n = 0-3. Each V¹ is preferably independently selected from the group consisting of substituted and unsubstituted C₁-C₆ alkyl, C₃-C₁₀ cycloalkyl, C₆-C₁₀ aryl, -NO₂, -NH₂, -C(O)-C₁₋₆ alkyl, -C(O)N-(C₁₋₆ alkyl)₂, -C(O)NH-C₁₋₆ alkyl, -O-C₁₋₆ alkyl, -NHC(O)H, -NHC(O)OH, -N(C₁₋₆ alkyl), C(O)-C₁₋₆ alkyl, -N-(C₁₋₆ alkyl)C(O)-C₁₋₆ alkyl, -OC(O)NH-C₆ -C₁₄ aryl, -NHC(O)-C₁₋₆alkyl, -OC(O)NH-C-NHC(O)O-C₁₋₆alkyl, -S(O)-C₁₋₆ alkyl and -SO₂-C₁₋₆ alkyl. Where V¹ is a substituted group, it is preferably substituted by one or more halogen, up to per-halosubstitution.

Most preferably, R² is selected from substituted and unsubstituted phenyl or pyridinyl groups, where the substituents are halogen and Wₙ (n = 0-3).

W is selected from the group consisting of -NO₂, -C₁₋₃ alkyl, -NH(O)CH₃, -CF₃, -OCH₃, -F, -Cl, -NH₂, -OC(O)NH, -SO₂CH₃, pyridinyl, phenyl, up to per-halosubstituted phenyl and C₁-C₆ alkyl substituted phenyl.

The present invention is also directed to pharmaceutically acceptable salts of formula I. Suitable pharmaceutically acceptable salts are well known to those skilled in the art and include basic salts of inorganic and organic acids, such as hydrochloric acid, hydrobromic acid, sulphuric acid, phosphoric acid, methanesulphonic acid, sulphonic acid, acetic acid, trifluoroacetic acid, malic acid, tartaric acid, citric acid, lactic acid, oxalic acid, succinic acid, fumaric acid, maleic acid, benzoic acid, salicylic acid, phenylacetic acid, and mandelic acid.

In addition, pharmaceutically acceptable salts include acid salts of inorganic bases, such as salts containing alkaline cations (e.g., Li⁺ Na⁺ or K⁺), alkaline earth cations (e.g., Mg⁺², Ca⁺² or Ba⁺²), the ammonium cation, as well as acid salts of organic bases, including aliphatic and aromatic substituted ammonium, and quaternary ammonium cations such as those arising from protonation or peralkylation of triethylamine, *N,N*-diethylamine, *N,N*-dicyclohexylamine, pyridine, *N,N-*dimethylaminopyridine (DMAP), 1,4-diazabiclo[2.2.2]octane (DABCO), 1,5-diazabicyclo[4.3.0]non-5-ene (DBN) and 1,8-diazabicyclo[5.4.0]undec-7-ene (DBU).

A number of the compounds of Formula I possess asymmetric carbons and can therefore exist in racemic and optically active forms. Methods of separation of enantiomeric and diastereomeric mixtures are well known to one skilled in the art. The present invention encompasses any isolated racemic or optically active form of compounds described in Formula I which possess p38 kinase inhibitory activity.

The compounds of Formula I may be prepared by use of known chemical reactions and procedures. Nevertheless, the following general preparative methods are presented to aid one of skill in the art in synthesizing the inhibitors, with more detailed particular examples being presented in the experimental section describing the working examples.

### General Preparative Methods

The compounds of Formula I may be prepared by the use of known chemical reactions and procedures, some from starting materials which are commercially available. Nevertheless, general preparative methods are provided below to aid one skilled in the art in synthesizing these compounds, with more detailed examples being provided in the Experimental section which follows.

Heterocyclic amines may be synthesized utilizing known methodology (Katritzky, et al. *Comprehensive Heterocyclic Chemistry;* Permagon Press: Oxford, UK (1984). March. *Advanced Organic Chemistry,* 3^{rd} Ed.; John Wiley: New York (1985)). For example, as shown in Scheme I, 5-aminopyrazoles substituted at the *N*-1 position with either aryl or heteroaryl moieties may be synthesized by the reaction of an α-cyanoketone (**2**) with the appropriate aryl- or heteroaryl hydrazine (**3**, R²=aryl or heteroaryl). Cyanoketone **2**, in turn, is available from the reaction of acetamidate ion with an appropriate acyl derivative, such as an ester, an acid halide, or an acid anhydride. In cases where the R² moiety offers suitable anion stabilization, 2-aryl-and 2-heteroarylfurans may be synthesized from a Mitsunobu reaction of cyanoketone **2** with alcohol **5**, followed by base catalyzed cyclization of enol ether **6** to give furylamine **7**.

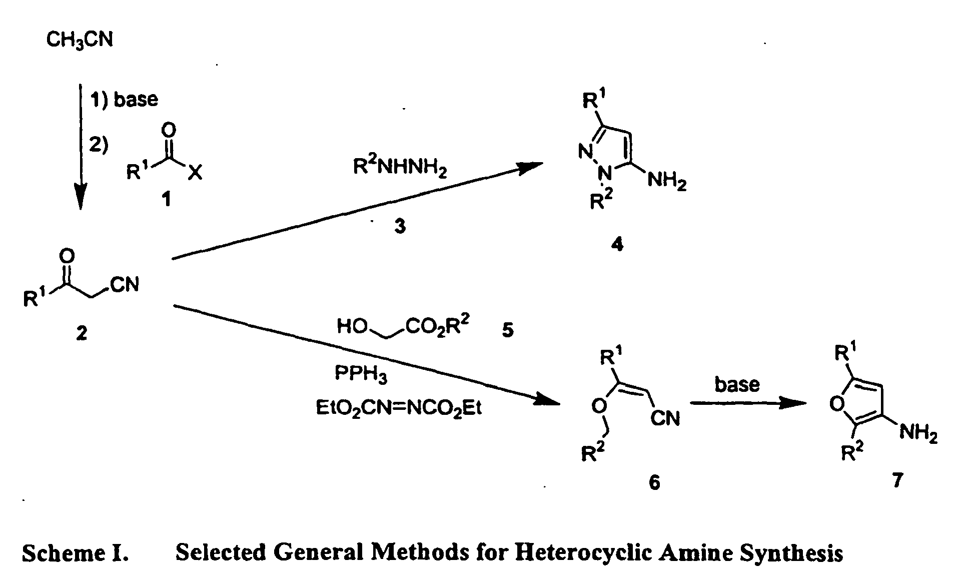

Substituted anilines may be generated using standard methods (March. *Advanced Organic Chemistry,* 3^{rd} Ed.; John Wiley: New York (1985). Larock. *Comprehensive Organic Transformations*; VCH Publishers: New York (1989)). As shown in Scheme II, aryl amines are commonly synthesized by reduction of nitroaryls using a metal catalyst, such as Ni, Pd, or Pt, and H₂ or a hydride transfer agent, such as formate, cyclohexadiene, or a borohydride (Rylander. *Hydrogenation Methods;* Academic Press: London, UK (1985)). Nitroaryls may also be directly reduced using a strong hydride source, such as LiAlH₄ (Seyden-Penne. *Reductions by the Alumino- and Borohydrides in Organic Synthesis;* VCH Publishers: New York (1991)), or using a zero valent metal, such as Fe, Sn or Ca, often in acidic media. Many methods exist for the synthesis of nitroaryls (March. *Advanced Organic Chemistry,* 3^{rd} Ed.; John Wiley: New York (1985). Larock. *Comprehensive Organic Transformations;* VCH Publishers: New York (1989)).

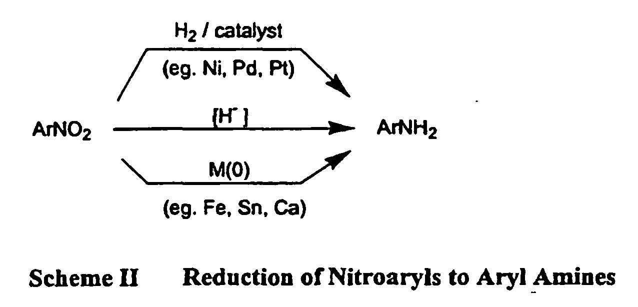

Nitroaryls are commonly formed by electrophilic aromatic nitration using HNO₃, or an alternative NO₂⁺ source. Nitro aryls may be further elaborated prior to reduction. Thus, nitroaryls substituted with 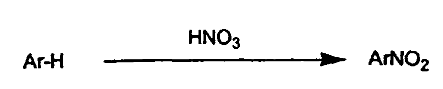 potential leaving groups (eg. F, Cl, Br, etc.) may undergo substitution reactions on treatment with nucleophiles, such as thiolate (exemplified in Scheme III) or phenoxide. Nitroaryls may also undergo Ullman-type coupling reactions (Scheme III).

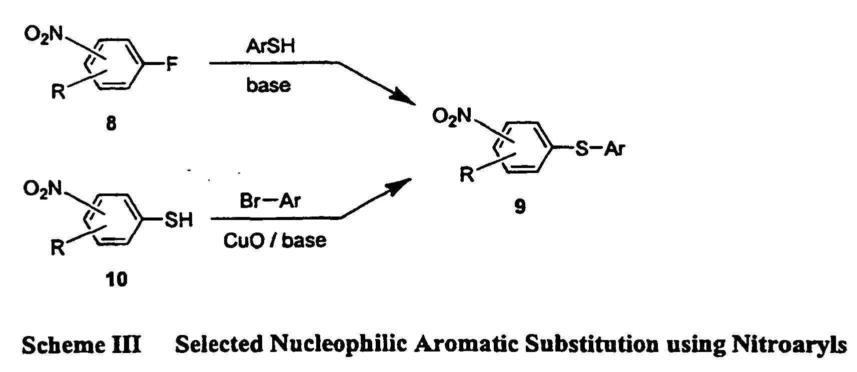

As shown in Scheme IV, urea formation may involve reaction of a heteroaryl isocyanate (**12**) with an aryl amine (**11**). The heteroaryl isocyanate may be synthesized from a heteroaryl amine by treatment with phosgene or a phosgene equivalent, such as trichloromethyl chloroformate (diphosgene), bis(trichloromethyl) carbonate (triphosgene), or *N,N'*-carbonyldiimidazole (CDI). The isocyanate may also be derived from a heterocyclic carboxylic acid derivative, such as an ester, an acid halide or an anhydride by a Curtius-type rearrangement. Thus, reaction of acid derivative **16** with an azide source, followed by rearrangement affords the isocyanate. The corresponding carboxylic acid (**17**) may also be subjected to Curtius-type rearrangements using diphenylphosphoryl azide (DPPA) or a similar reagent. A urea may also be generated from the reaction of an aryl isocyanate (**15**) with a heterocyclic amine. 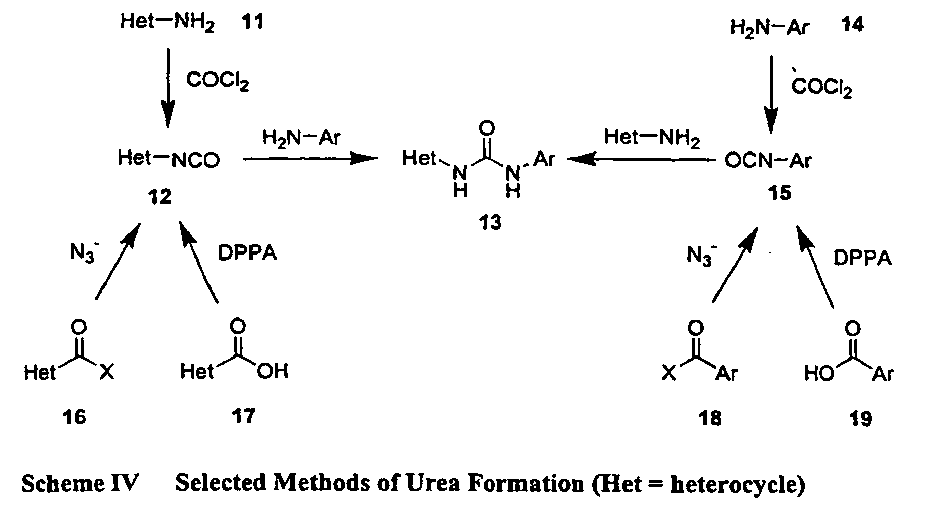 Finally, ureas may be further manipulated using methods familiar to those skilled in the art. For example, 2-aryl and 2-heteroarylthienyl ureas are available from the corresponding 2-halothienyl urea through transition metal mediated cross coupling reactions (exemplified with 2-bromothiophene **25**, Scheme V). Thus, reaction of nitrile **20** with an α-thioacetate ester gives 5-substituted-3-amino-2-thiophenecarboxylate **21** (Ishizaki et al. JP 6025221). Decarboxylation of ester **21** may be achieved by protection of the amine, for example as the *tert*-butoxy (BOC) carbamate (**22**), followed by saponification and treatment with acid. When BOC protection is used, decarboxylation may be accompanied by deprotection giving the substituted 3-thiopheneammonium salt **23**. Alternatively, ammonium salt **23** may be directly generated through saponification of ester **21** followed by treatment with acid. Following urea formation as described above, bromination affords penultimate halothiophene **25**. Palladium mediated cross coupling of thiophene **25** with an appropriate-tributyl- or trimethyltin (R²= aryl or heteroaryl) then affords the desired 2-aryl- or 2-heteroarylthienyl urea. 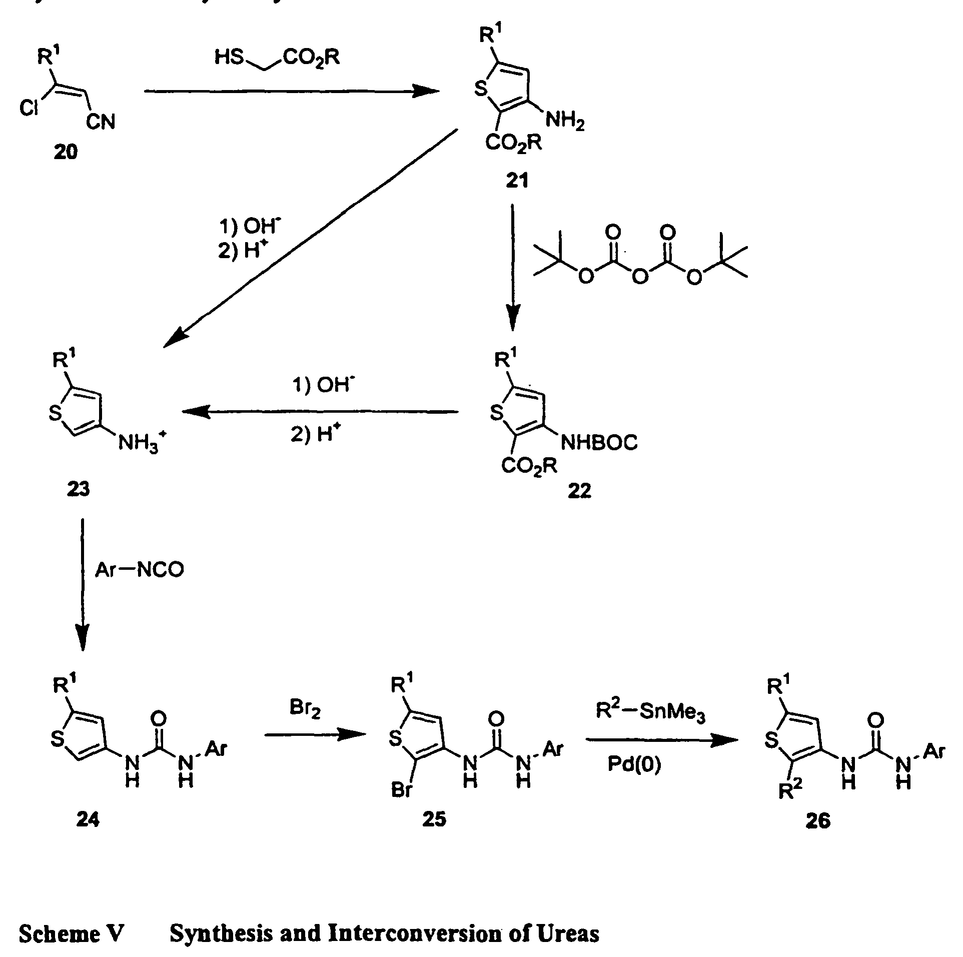 The invention also includes pharmaceutical compositions including a compound of Formula I, and a physiologically acceptable carrier.

The compounds may be administered orally, topically, parenterally, by inhalation or spray or vaginally, rectally or sublingually in dosage unit formulations. The term 'administration by injection' includes intravenous, intramuscular, subcutaneous and parenteral injections, as well as use of infusion techniques. Dermal administration may include topical application or transdermal administration. One or more compounds may be present in association with one or more non-toxic pharmaceutically acceptable carriers and if desired other active ingredients.

Compositions intended for oral use may be prepared according to any suitable method known to the art for the manufacture of pharmaceutical compositions. Such compositions may contain one or more agents selected from the group consisting of diluents, sweetening agents, flavoring agents, coloring agents and preserving agents in order to provide palatable preparations. Tablets contain the active ingredient in admixture with non-toxic pharmaceutically acceptable excipients which are suitable for the manufacture of tablets. These excipients may be, for example, inert diluents, such as calcium carbonate, sodium carbonate, lactose, calcium phosphate or sodium phosphate; granulating and disintegrating agents, for example, corn starch, or alginic acid; and binding agents, for example magnesium stearate, stearic acid or talc. The tablets may be uncoated or they may be coated by known techniques to delay disintegration and adsorption in the gastrointestinal tract and thereby provide a sustained action over a longer period. For example, a time delay material such as glyceryl monostearate or glyceryl distearate may be employed. These compounds may also be prepared in solid, rapidly released form.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

Aqueous suspensions containing the active materials in admixture with excipients suitable for the manufacture of aqueous suspensions may also be used. Such excipients are suspending agents, for example sodium carboxymethylcellulose, methylcellulose, hydroxypropyl-methylcellulose, sodium alginate, polyvinylpyrrolidone, gum tragacanth and gum acacia; dispersing or wetting agents may be a naturally-occurring phosphatide, for example, lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, *p*-hydroxybenzoate, one or more coloring agents, one or more flavoring agents, and one or more sweetening agents, such as sucrose or saccharin.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example, sweetening, flavoring and coloring agents, may also be present.

The compounds may also be in the form of non-aqueous liquid formulations, e.g., oily suspensions which may be formulated by suspending the active ingredients in a vegetable oil, for example arachis oil, olive oil, sesame oil or peanut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide palatable oral preparations. These compositions may be preserved by the addition of an anti-oxidant such as ascorbic acid.

Pharmaceutical compositions of the invention may also be in the form of oil-in-water emulsions. The oil phase may be a vegetable oil, for example olive oil or arachis oil, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavoring agents.

Syrups and elixirs may be formulated with sweetening agents, for example glycerol, propylene glycol, sorbitol or sucrose. Such formulations may also contain a demulcent, a preservative and flavoring and coloring agents.

The compounds may also be administered in the form of suppositories for rectal or vaginal administration of the drug. These compositions can be prepared by mixing the drug with a suitable non-irritating excipient which is solid at ordinary temperatures but liquid at the rectal or vaginal temperature and will therefore melt in the rectum or vagina to release the drug. Such materials include cocoa butter and polyethylene glycols.

Compounds of the invention may also be administrated transdermally using methods known to those skilled in the art (see, for example: Chien; "Transdermal Controlled Systemic Medications"; Marcel Dekker, Inc.; 1987. Lipp et al. WO94/04157 3Mar94). For example, a solution or suspension of a compound of Formula I in a suitable volatile solvent optionally containing penetration enhancing agents can be combined with additional additives known to those skilled in the art, such as matrix materials and bacteriocides. After sterilization, the resulting mixture can be formulated following known procedures into dosage forms. In addition, on treatment with emulsifying agents and water, a solution or suspension of a compound of Formula I may be formulated into a lotion or salve.

Suitable solvents for processing transdermal delivery systems are known to those skilled in the art, and include lower alcohols such as ethanol or isopropyl alcohol, lower ketones such as acetone, lower carboxylic acid esters such as ethyl acetate, polar ethers such as tetrahydrofuran, lower hydrocarbons such as hexane, cyclohexane or benzene, or halogenated hydrocarbons such as dichloromethane, chloroform, trichlorotrifluoroethane, or trichlorofluoroethane. Suitable solvents may also include mixtures of one or more materials selected from lower alcohols, lower ketones, lower carboxylic acid esters, polar ethers, lower hydrocarbons, halogenated hydrocarbons.

Suitable penetration enhancing materials for transdermal delivery system are known to those skilled in the art, and include, for example, monohydroxy or polyhydroxy alcohols such as ethanol, propylene glycol or benzyl alcohol, saturated or unsaturated C₈-C₁₈ fatty alcohols such as lauryl alcohol or cetyl alcohol, saturated or unsaturated C₈-C₁₈ fatty acids such as stearic acid, saturated or unsaturated fatty esters with up to 24 carbons such as methyl, ethyl, propyl, isopropyl, n-butyl, sec-butyl, isobutyl, tertbutyl or monoglycerin esters of acetic acid, capronic acid, lauric acid, myristinic acid, stearic acid, or palmitic acid, or diesters of saturated or unsaturated dicarboxylic acids with a total of up to 24 carbons such as diisopropyl adipate, diisobutyl adipate, diisopropyl sebacate, diisopropyl maleate, or diisopropyl fumarate. Additional penetration enhancing materials include phosphatidyl derivatives such as lecithin or cephalin, terpenes, amides, ketones, ureas and their derivatives, and ethers such as dimethyl isosorbid and diethyleneglycol monoethyl ether. Suitable penetration enhancing formulations may also include mixtures of one or more materials selected from monohydroxy or polyhydroxy alcohols, saturated or unsaturated C₈-C₁₈ fatty alcohols, saturated or unsaturated C₈-C₁₈ fatty acids, saturated or unsaturated fatty esters with up to 24 carbons, diesters of saturated or unsaturated discarboxylic acids with a total of up to 24 carbons, phosphatidyl derivatives, terpenes, amides, ketones, ureas and their derivatives, and ethers.

Suitable binding materials for transdermal delivery systems are known to those skilled in the art and include polyacrylates, silicones, polyurethanes, block polymers, styrenebutadiene coploymers, and natural and synthetic rubbers. Cellulose ethers, derivatized polyethylenes, and silicates may also be used as matrix components. Additional additives, such as viscous resins or oils may be added to increase the viscosity of the matrix.

For all regimens of use disclosed herein for compounds of Formula I, the daily oral dosage regimen will preferably be from 0.01 to 200 mg/Kg of total body weight. The daily dosage for administration by injection, including intravenous, intramuscular, subcutaneous and parenteral injections, and use of infusion techniques will preferably be from 0.01 to 200 mg/Kg of total body weight. The daily vaginal dosage regimen will preferably be from 0.01 to 200 mg/Kg of total body weight. The daily rectal dosage regimen will preferably be from 0.01 to 200 mg/Kg of total body weight. The daily topical dosage regimen will preferably be from 0.1 to 200 mg administered between one to four times daily. The transdermal concentration will preferably be that required to maintain a daily dose of from 0.01 to 200 mg/Kg. The daily inhalation dosage regimen will preferably be from 0.01 to 10 mg/Kg of total body weight.

It will be appreciated by those skilled in the art that the particular method of administration will depend on a variety of factors, all of which are considered routinely when administering therapeutics. It will also be understood, however, that the specific dose level for any given patient will depend upon a variety of factors, including, the activity of the specific compound employed, the age of the patient, the body weight of the patient, the general health of the patient, the gender of the patient, the diet of the patient, time of administration, route of administration, rate of excretion, drug combinations, and the severity of the condition undergoing therapy. It will be further appreciated by one skilled in the art that the optimal course of treatment, ie, the mode of treatment and the daily number of doses of a compound of Formulae I or a pharmaceutically acceptable salt thereof given for a defined number of days, can be ascertained by those skilled in the art using conventional course of treatment tests.

The entire disclosure of all applications, patents and publications cited above and below are hereby incorporated by reference, including provisional application (Attorney Docket Number BAYER 12V1), filed on December 22, 1997, as SN 08/995,751, and converted on December 22, 1998.

The following examples are for illustrative purposes only and are not intended, nor should they be construed to limit the invention in any way.

### EXAMPLES

All reactions were performed in flame-dried or oven-dried glassware under a positive pressure of dry argon or dry nitrogen, and were stirred magnetically unless otherwise indicated. Sensitive liquids and solutions were transferred via syringe or cannula, and introduced into reaction vessels through rubber septa. Unless otherwise stated, the term 'concentration under reduced pressure' refers to use of a Buchi rotary evaporator at approximately 15 mmHg.

All temperatures are reported uncorrected in degrees Celsius (°C). Unless otherwise indicated, all parts and percentages are by weight.

Commercial grade reagents and solvents were used without further purification. Thin-layer chromatography (TLC) was performed on Whatman^{®} pre-coated glass-backed silica gel 60A F-254 250 µm plates. Visualization of plates was effected by one or more of the following techniques: (a) ultraviolet illumination, (b) exposure to iodine vapor, (c) immersion of the plate in a 10% solution of phosphomolybdic acid in ethanol followed by heating, (d) immersion of the plate in a cerium sulfate solution followed by heating, and/or (e) immersion of the plate in an acidic ethanol solution of 2,4-dinitrophenylhydrazine followed by heating. Column chromatography (flash chromatography) was performed using 230-400 mesh EM Science^{®} silica gel.

Melting points (mp) were determined using a Thomas-Hoover melting point apparatus or a Mettler FP66 automated melting point apparatus and are uncorrected. Proton (¹H) nuclear magnetic resonance (NMR) spectra were measured with a General Electric GN-Omega 300 (300 MHz) spectrometer with either Me₄Si (δ 0.00) or residual protonated solvent (CHCl₃ δ 7.26; MeOH δ 3.30; DMSO δ 2.49) as standard. Carbon (¹³C) NMR spectra were measured with a General Electric GN-Omega 300 (75 MHz) spectrometer with solvent (CDCl₃ δ 77.0; MeOD-d₃; δ 49.0; DMSO-d₆ δ 39.5) as standard. Low resolution mass spectra (MS) and high resolution mass spectra (HRMS) were either obtained as electron impact (EI) mass spectra or as fast atom bombardment (FAB) mass spectra. Electron impact mass spectra (EI-MS) were obtained with a Hewlett Packard 5989A mass spectrometer equipped with a Vacumetrics Desorption Chemical Ionization Probe for sample introduction. The ion source was maintained at 250 °C. Electron impact ionization was performed with electron energy of 70 eV and a trap current of 300 µA. Liquid-cesium secondary ion mass spectra (FAB-MS), an updated version of fast atom bombardment were obtained using a Kratos Concept 1-H spectrometer. Chemical ionization mass spectra (CI-MS) were obtained using a Hewlett Packard MS-Engine (5989A) with methane as the reagent gas (1x10⁻⁴ torr to 2.5x10⁻⁴ torr). The direct insertion desorption chemical ionization (DCI) probe (Vaccumetrics, Inc.) was ramped from 0-1.5 amps in 10 sec and held at 10 amps until all traces of the sample disappeared (~1-2 min). Spectra were scanned from 50-800 amu at 2 sec per scan. HPLC - electrospray mass spectra (HPLC ES-MS) were obtained using a Hewlett-Packard 1100 HPLC equipped with a quaternary pump, a variable wavelength detector, a C-18 column, and a Finnigan LCQ ion trap mass spectrometer with electrospray ionization. Spectra were scanned from 120-800 amu using a variable ion time according to the number of ions in the source. Gas chromatography - ion selective mass spectra (GC-MS) were obtained with a Hewlett Packard 5890 gas chromatograph equipped with an HP-1 methyl silicone column (0.33 mM coating; 25 m x 0.2 mm) and a Hewlett Packard 5971 Mass Selective Detector (ionization energy 70 eV).

Elemental analyses were conducted by Robertson Microlit Labs, Madison NJ. All ureas displayed NMR spectra, LRMS and either elemental analysis or HRMS consistant with assigned structures.

### List of Abbreviations and Acronyms:

- AcOH: acetic acid
- anh: anhydrous
- BOC: *tert*-butoxycarbonyl
- cone: concentrated
- dec: decomposition
- DMPU: 1,3-dimethyl-3,4,5,6-tetrahydro-2(1H)-pyrimidinone
- DMF: *N,N*-dimethylformamide
- DMSO: dimethylsulfoxide
- DPPA: diphenylphosphoryl azide
- EtOAc: ethyl acetate
- EtOH: ethanol (100%)
- Et₂O: diethyl ether
- Et₃N: triethylamine
- *m*-CPBA: 3-chloroperoxybenzoic acid
- MeOH: methanol
- pet. ether: petroleum ether (boiling range 30-60 °C)
- THF: tetrahydrofuran
- TFA: trifluoroacetic acid
- Tf: trifluoromethanesulfonyl

### A. General Methods for Synthesis of Heterocyclic Amines

### A1. General Procedure for the Preparation of N¹-Aryl-5-aminopyrazoles

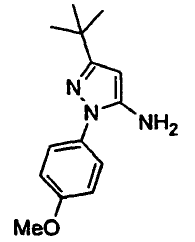

***N***^{**1**}**-(4-Methoxyphenyl)-5-amino-3-*tert*-butylpyrazole:** A mixture of 4-methoxyphenylhydrazine hydrochloride (3.5 g), 4,4-dimethyl-3-oxopentanenitrile (2.5 g), EtOH (30 mL), and AcOH (1 mL) was heated at the reflux temperature for 3 h, cooled to room temp., and poured into a mixture of Et₂O (100 mL) and a 10% Na₂CO₃ solution (100 mL). The organic layer was washed with a saturated NaCl solution, dried (MgSO₄) and concentrated under reduced pressure. The solid residue was washed with pentane to afford the desired pyrazole as a pale brown solid. (4.25g): ¹H-NMR (DMSO-d₆) δ 1.18 (s, 9H); 3.78 (s, 3H); 5.02 (br s, 2H); 5.34 (s, 1H); 6.99 (d, *J*=8 Hz, 2H); 7.42 (d, *J*=8 Hz, 2H).

### A2. General Method for the Mitsunobu-Based Synthesis of 2-Aryl-3-aminofurans

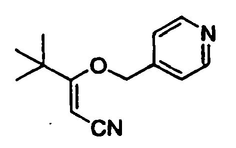

**Step 1. 4,4-Dimethyl-3-(4-pyridinylmethoxy)-2-pentenenitrile:** A solution of triphenylphosphine (2.93 g, 11.2 mmol) in anh THF (50 mL) was treated with diethyl azodicarboxylate (1.95 g, 11.2 mmol) and 4-pyridinylmethanol (1.22 g, 11.2 mmol), then stirred for 15 min. The resulting white slurry was treated with 4,4-dimethyl-3-oxopentanenitrile (1.00 g, 7.99 mmol), then stirred for 15 min. The reaction mixture was concentrated under reduced pressure. The residue was purified by column chromatography (30% EtOAc/70% hexane) to give the desired nitrile as a yellow solid (1.83 g, 76%): TLC (20% EtOAc/80% hexane) R_{*f*} 0.13; ¹H-NMR (CDCl₃) δ 1.13 (s, 9H), 4.60 (s, 1H), 5.51 (s, 2H), 7.27 (d, *J*=5.88 Hz, 2H), 8.60 (d, *J*=6.25 Hz, 2H); ¹³C-NMR (CDCl₃) δ 27.9 (3C), 38.2, 67.5, 70.8, 117.6, 121.2 (2C), 144.5, 149.9 (2C), 180.7; CI-MS *m*/*z* (rel abundance) 217 ((M+H)⁺, 100%).

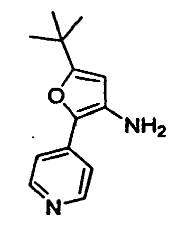

**Step 2. 3-Amino-2-(4-pyridinyl)-5-*tert*-butylfuran:** A solution of 4,4-dimethyl-3-(4-pyridinylmethoxy)-2-pentenenitrile (1.55 g, 7.14 mmol) in anh DMSO (75 mL) was treated with potassium *tert-*butoxide (0.88 g, 7.86 mmol) and stirred at room temp for 10 min. The resulting mixture was treated with EtOAc (300 mL), then sequentially washed with water (2 x 200 mL) and a saturated NaCl solution (100 mL). Combined aqueous phases were back-extracted with EtOAc (100 mL). The combined organic phases were dried (Na₂SO₄) and concentrated under reduced pressure. The residue was purified by column chromatography (gradient from 30% EtOAc/70% hexane to 100% EtOAc) to give the desired product as an orange oil (0.88 g, 57%): TLC (40% EtOAc/60% hexane) R_{*f*} 0.09; ¹H-NMR (CDCl₃) δ 1.28 (s, 9H), 3.65 (br s, 2H), 5.79 (s, 1H), 7.30 (d, *J*=6.25 Hz, 2H), 8.47 (d, *J*=6.25 Hz, 2H); EI-MS *m*/*z* (rel abundance) 216 (M⁺, 30%).

### A3. Synthesis 3-Amino-5-alkylthiophenes from N-BOC 3-Amino-5-alkyl-2-thiophenecarboxylate esters

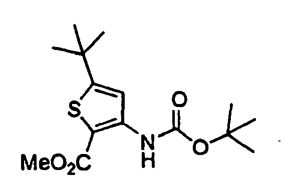

**Step 1. Methyl 3-(*tert*-Butoxycarbonylamino)-5-*tert*-butyl-2-thiophenecarboxylate:** To a solution of methyl 3-amino-5-*tert*-butyl-2-thiophenecarboxylate (150 g, 0.70 mol) in pyridine (2.8 L) at 5 °C was added di-*tert*-butyl dicarbonate (171.08 g, 0.78 mol, 1.1 equiv) and *N,N*-dimethylaminopyridine (86 g, 0.70 mol, 1.00 equiv) and the resulting mixture was stirred at room temp for 7 d. The resulting dark solution was concentrated under reduced pressure (approximately 0.4 mmHg) at approximately 20 °C. The resulting red solids were dissolved in CH₂Cl₂ (3 L) and sequentially washed with a 1 M H₃PO₄ solution (2 x 750 mL), a saturated NaHCO₃ solution (800 mL) and a saturated NaCl solution (2 x 800 mL), dried (Na₂SO₄) and concentrated under reduced pressure. The resulting orange solids were dissolved in abs. EtOH (2 L) by warming to 49 °C, then treated with water (500 mL) to afford the desired product as an off-white solid (163 g, 74%): ¹H-NMR (CDCl₃) δ 1.38 (s, 9H), 1.51 (s, 9H), 3.84 (s, 3H), 7.68 (s, 1H), 9.35 (br s, 1H); FAB-MS *m*/*z* (rel abundance) 314 ((M+H)⁺, 45%).

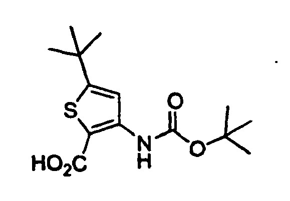

**Step 2. 3-(*tert*-Butoxytarbonylamino)-5-*tert*-butyl-2-thiophenecarboxylic Acid:** To a solution of methyl 3-(*tert*-butoxycarbonylamino)-5-*tert-*butyl-2-thiophenecarboxylate (90.0 g, 0.287 mol) in THF (630 mL) and MeOH (630 mL) was added a solution of NaOH (42.5 g, 1.06 mL) in water (630 mL). The resulting mixture was heated at 60 °C for 2 h, concentrated to approximately 700 mL under reduced pressure, and cooled to 0 °C. The pH was adjusted to approximately 7 with a 1.0 N HCl solution (approximately 1 L) while maintaining the internal temperature at approximately 0 °C. The resulting mixture was treated with EtOAc (4 L). The pH was adjusted to approximately 2 with a 1.0 N HCl solution (500 mL). The organic phase was washed with a saturated NaCl solution (4 x 1.5 L), dried (Na₂SO₄), and concentrated to approximately 200 mL under reduced pressure. The residue was treated with hexane (1 L) to form a light pink (41.6 g). Resubmission of the mother liquor to the concentration-precipitation protocol afforded additional product (38.4 g, 93% total yield): ¹H-NMR (CDCl₃) δ 1.94 (s, 9H), 1.54 (s, 9H), 7.73 (s, 1H), 9.19 (br s, 1H); FAB-MS *m*/*z* (rel abundance) 300 ((M+H)⁺, 50%).

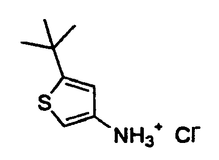

**Step 3. 5-*tert*-Butyl-3-thiopheneammonium Chloride:** A solution of 3-(*tert-*butoxycarbonylamino)-5-*tert*-butyl-2-thiophenecarboxylic acid (3.0 g, 0.010 mol) in dioxane (20 mL) was treated with an HCl solution (4.0 M in dioxane, 12.5 mL, 0.050 mol, 5.0 equiv), and the resulting mixture was heated at 80 °C for 2 h. The resulting cloudy solution was allowed to cool to room temp forming some precipitate. The slurry was diluted with EtOAc (50 mL) and cooled to -20 °C. The resulting solids were collected and dried overnight under reduced pressure to give the desired salt as an off-white solid (1.72 g, 90%): ¹H-NMR (DMSO-d₆) δ 1.31 (s, 9H), 6.84 (d, *J*=1.48 Hz, 1H), 7.31 (d, *J*=1.47 Hz, 1H), 10.27 (br s, 3H).

### B. General Methods for Synthesis of Substituted Anilines

### B1. General Method for Substituted Aniline Synthesis via Nucleophilic Aromatic Substitution using a Halopyridine

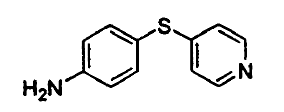

**3-(4-Pyridinylthio)aniline:** To a solution of 3-aminothiophenol (3.8 mL, 34 mmoles) in anh DMF (90mL) was added 4-chloropyridine hydrochloride (5.4 g, 35.6 mmoles) followed by K₂CO₃ (16.7 g, 121 mmoles). The reaction mixture was stirred at room temp. for 1.5 h, then diluted with EtOAc (100 mL) and water (100mL). The aqueous layer was back-extracted with EtOAc (2 x 100 mL). The combined organic layers were washed with a saturated NaCl solution (100 mL), dried (MgSO₄), and concentrated under reduced pressure. The residue was filtered through a pad of silica (gradient from 50% EtOAc/50% hexane to 70% EtOAc/30% hexane) and the resulting material was triturated with a Et₂O/hexane solution to afford the desired product (4.6 g, 66%): TLC (100 % ethyl acetate) R_{*f*}0.29; ¹H-NMR (DMSO-d₆) δ 5.41 (s, 2H), 6.64-6.74 (m, 3H), 7.01 (d, J=4.8, 2H), 7.14 (t, J=7.8 Hz, 1H), 8.32 (d, J=4.8, 2H).

### C. General Methods of Urea Formation

### C1a. Reaction of a Heterocyclic Amine with an Aryl Isocyanate

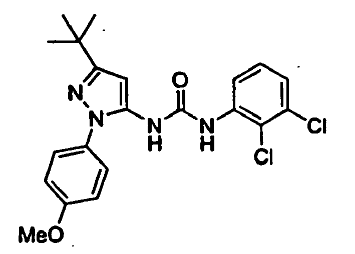

***N*-(1-(4-Methoxyphenyl)-3-*tert*-butyl-5-pyrazolyl)-*N'*-(2,3-dichlorophenyl)urea:** To a stirring solution of 1-(4-methoxyphenyl)-3-*tert*-butyl-5-aminopyrazole (0.342 g, 1.39 mmol) in anh toluene (9 mL) was added 2,3-dichlorophenyl isocyanate (0.276 mL, 2.09 mmol). The solution was sealed and stirred in the dark for 96 h at 60 °C. After this time, the reaction mixture was diluted with EtOAc (200 mL). The resulting mixture was sequentially washed with a 1 M HCl solution (2 x 125 mL) and a saturated NaCl solution (50 mL), dried (MgSO₄), and concentrated under reduced pressure. The residue was purified by column chromatography (20% EtOAc/80% hexane) to give the product as a white solid (0.335 g, 56%): TLC (20% EtOAc/80% hexane) R_{*f*} 0.22; ¹H NMR (DMSO-d₆) δ 1.24 (s, 9H), 3.79 (s, 3H), 6.33 (s, 1H), 7.05 (d, *J*=9 Hz, 2H), 7.28 (m, 2H), 7.38 (d, *J*=9 Hz, 2H), 8.05 (dd, *J*=3, 6 Hz, 1H), 8.75 (s, 1H), 9.12 (s, 1H); FAB-MS *m*/*z* 433 ((M+H)⁺).

### C1b. Reaction of a Heterocyclic Amine with an Aryl Isocyanate

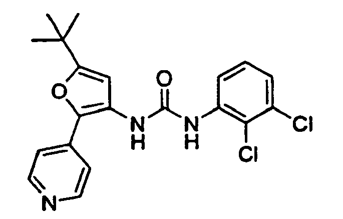

***N*-(2-(4-Pyridinyl)-5-*tert*-butyl-3-furyl)-*N'*-(2,3-dichlorophenyl)urea:** A solution of 3-amino-2-(4-pyridinyl)-5-*tert*-butylfuran (Method A2; 0.10 g, 0.46 mmol) and 2,3-dichlorophenyl isocyanate (0.13 g, 0.69 mmol) in CH₂Cl₂ was stirred at room temp. for 2 h, then was treated with 2-(dimethylamino)ethylamine (0.081 g, 0.92 mmol) and stirred for an additional 30 min. The resulting mixture was diluted with EtOAc (50 mL), then was sequentially washed with a 1 N HCl solution (50 mL), a saturated NaHCO₃ solution (50 mL) and a saturated NaCl solution (50 mL), dried (Na₂SO₄), and concentrated under reduced pressure. The residue was purified using column chromatography (gradient from 10% EtOAc/90% hexane to 40% EtOAc/60% hexane) to give the desired compound as a white solid (0.12 g, 63%): mp 195-198 °C; TLC (60% EtOAc/40% hexane ) R_{*f*}0.47; ¹H NMR (DMSO-d₆) δ 1.30 (s, 9H); 6.63 (s, 1H); 7.30-7.32 (m, 2H), 7.58 (dm, *J*=6.62 Hz, 2H), 8.16 (dd, *J*=2.57, 6.99 Hz, 1H), 8.60 (dm, *J*=6.25 Hz, 2H), 8.83 (s, 1H), 9.17 (s, 1H); ¹³C NMR (DMSO-d₆) δ 28.5 (3C), 32.5, 103.7, 117.3 (2C), 119.8, 120.4, 123.7, 125.6, 128.1, 131.6, 135.7, 136.5, 137.9, 150.0 (2C), 152.2, 163.5; CI-MS *m*/*z* (rel abundance) 404 ((M+H)⁺, 15%), 406 ((M+H+2)⁺, 8%).

### C1c. Reaction of a Heterocyclic Amine with an Isocyanate

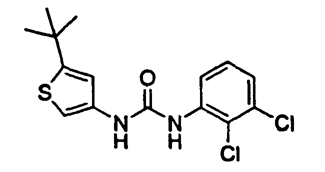

***N*-(5-*tert*-Butyl-3-thienyl)-*N'*-(2,3-dichlorophenyl)urea:** Pyridine (0.163 mL, 2.02 mmol) was added to a slurry of 5-*tert*-butylthiopheneammonium chloride (Method A4c; 0.30 g, 1.56 mmol) and 2,3-dichlorophenyl isocyanate (0.32 mL, 2.02 mmol) in CH₂Cl₂ (10 mL) to clarify the mixture and the resulting solution was stirred at room temp. overnight. The reaction mixture was then concentrated under reduced pressure and the residue was separated between EtOAc (15 mL) and water (15 mL). The organic layer was sequentially washed with a saturated NaHCO₃ solution (15 mL), a 1N HCl solution (15 mL) and a saturated NaCl solution (15 mL), dried (Na₂SO₄), and concentrated under reduced pressure. A portion of the residue was by preparative HPLC (C-18 column; 60% acetonitrile/40% water/0.05% TFA) to give the desired urea (0.180 g, 34%): mp 169-170 °C; TLC (20% EtOAc/80% hexane) R_{*f*} 0.57; ¹H-NMR (DMSO-d₆) δ 1.31 (s, 9H), 6.79 (s, 1H), 7.03 (s, 1H), 7.24-7.33 (m, 2H), 8.16 (dd, *J*=1.84, 7.72 Hz, 1H), 8.35 (s, 1H), 9.60 (s, 1H); ¹³C-NMR (DMSO-d₆) δ 31.9 (3C), 34.0, 103.4, 116.1, 119.3, 120.0, 123.4, 128.1, 131.6, 135.6, 138.1, 151.7, 155.2; FAB-MS *m*/*z* (rel abundance) 343 ((M+H)⁺, 83%), 345 ((M+H+2)⁺, 56%), 347 ((M+H+4)⁺, 12%).

### C2 Reaction of Substituted Aniline with N,N'-Carbonyldiimidazole Followed by Reaction with a Heterocyclic Amine

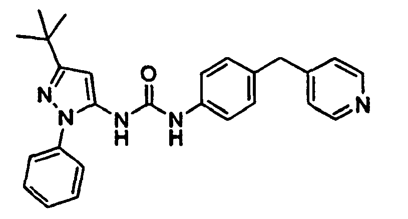

***N*-(1-Phenyl-3-*tert*-butyl-5-pyrazolyl)-*N'*-(4-(4-pyridinylmethyl)phenyl)urea:** A solution of 4-(4-pyridinylmethyl)aniline (0.25 g, 1.38 mmol) and *N,N'-*carbonyldiimidazole (0.23 g, 1.42 mmol) in CH₂Cl₂ 11 mL) at room temp. was stirred for 2 h, then treated with 5-amino-1-phenyl-3-*tert*-butyl-5-pyrazole (0.30 g, 1.38 mmol) and the resulting mixture was stirred at 50 °C overnight. The reaction mixture was diluted with EtOAc (25 mL), then sequentially washed with water (30 mL) and a saturated NaCl solution (30 mL), dried (MgSO₄), and concentrated under reduced pressure. The residue was purified by column chromatography (gradient from 100% CH₂Cl₂ to 30% acetone/70% CH₂Cl₂) and the resulting material was recrystallized (EtOAc/Et₂O) to give the desired product complexed with 0.25 equiv H₂O (0.30 g): TLC (60% acetone/40% CH₂Cl₂) R_{*f*}0.56; ¹H-NMR (DMSO-d₆) δ 1.25 (s, 9H); 3.86 (s, 2H), 6.34 (s, 1H), 7.11 (d, *J*=8.82 Hz, 2H), 7.19 (dm, *J*=6.25 Hz, 2H), 7.31 (d, *J*=1.84 Hz, 2H), 7.35-7.51 (m, 5 H), 8.34 (s, 1H), 8.42 (dm, *J*=5.98 Hz, 2H), 8.95 (s, 1H); FAB-MS *m*/*z* (rel abundance) 426 ((M+H)⁺, 100%).

### D. Interconversion of Ureas

### D1. General Method for Electrophylic Halogenation of Aryl Ureas

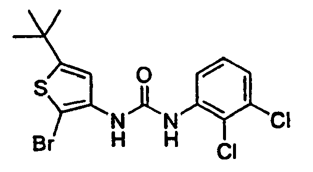

***N-*(2-Bromo-5-*tert*-butyl-3-thienyl)-*N'*-(2-3-dichlorophenyl)urea:** To a slurry of *N-*(5-*tert*-butyl-3-thienyl)-*N*'-(2,3-dichlorophenyl)urea (Method C1c; 3.00 g, 8.74 mmol) in CHCl₃ (200 mL) at room temp was slowly added a solution of Br₂ (0.46 mL, 1.7 mmol) in CHCl₃ (150 mL) via addition funnel over 2.5 h, causing the reaction mixture to become homogeneous. Stirring was continued 20 min after which TLC analysis indicated complete reaction. The reaction mixture was concentrated under reduced pressure, and the residue triturated (Et₂O/hexane) and the resulting solids were washed (hexane) to give the brominated product as a pink powder (3.45 g, 93%): mp 180-183 °C; TLC (10% EtOAc/90% hexane) R_{*f*} 0.68; ¹H NMR (DMSO-d₆) δ 1.28 (s, 9H), 7.27-7.31 (m, 2H), 7.33 (s, 1H), 8.11 (dd, *J*=3.3, 6.6 Hz, 1H), 8.95 (s, 1H), 9.12 (s, 1H); ¹³C NMR (DMSO-d₆) δ 31.5 (3C), 34.7, 91.1, 117.9, 120.1, 120.5, 123.8, 128.0, 131.6, 135.5, 137.9, 151.6, 155.3; FAB-MS *m*/*z* (rel abundance) 421 ((M+H)⁺, 7%), 423 (M+2+H)⁺, 10%).

### D2. General Method for Metal-Mediated Cross-Coupling Reactions with Halogen-Substituted Ureas

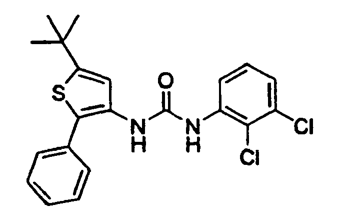

***N*-(2-Phenyl-5-*tert*-butyl-3-thienyl)-*N'*-(2,3-dichlorophenyl)urea:** To a solution of *N*-(3-(2-bromo-5-*tert*-butylthienyl)-*N'*-(2,3-dichlorophenyl)urea (0.50 g, 1.18 mmol) and phenyltrimethyltin (0.21 mL, 1.18 mmol) in DMF (15 mL) was added Pd(PPh₃)₂Cl₂ (0.082 g, 0.12 mmol), and the resulting suspension was heated at 80°C overnight. The reaction mixture was diluted with EtOAc (50 mL) and water (50 mL), and the organic layer sequentially washed with water (3 x 50 mL) and a saturated NaCl solution (50 mL), then dried (Na₂SO₄) and concentrated under reduced pressure. The residue was purified by MPLC (Biotage^{®}; gradient from 100% hexane to 5% EtOAc/95% hexane) followed by preparative HPLC (C-18 column; 70% CH₃CN/30% water/0.05% TFA). The HPLC fractions were concentrated under reduced pressure and the resulting aqueous mixture was extracted with EtOAc (2 x 50 mL). The combined organic layers were dried (Na₂SO₄) and concentrated under reduced pressure to give a gummy semi-solid, which was triturated with hexane to afford the desired product as a white solid (0.050 g, 10%): mp 171-173 °C; TLC (5% EtOAc/95% hexane) R_{*f*} 0.25; ¹H NMR (CDCl₃) δ 1.42 (s, 9H), 6.48 (br s, 1H), 7.01 (s, 1H), 7.10-7.18 (m, 2H), 7.26-7.30 (m, 1H), 7.36 (app t, *J*=7.72 Hz, 2H), 7.39 (br s, 1H), 7.50 (dm, *J*=6.99 Hz, 2H), 7.16 (dd, *J*=2.20, 7.72 Hz, 1H); ¹³C NMR (CDCl₃) δ 32.1 (3C), 34.8, 118.4, 118.8, 120.7, 121.1, 124.2, 127.7, 127.9, 128.2 (2C), 128.5, 129.0 (2C), 132.4, 132.5, 136.9, 153.1, 156.3; FAB-MS *m*/*z* (rel abundance) 419 ((M+H)⁺, 6%), 421 ((M+H+2)⁺, 4%).

### D3. General Methods of Reduction of Nitro-Containing Aryl Ureas

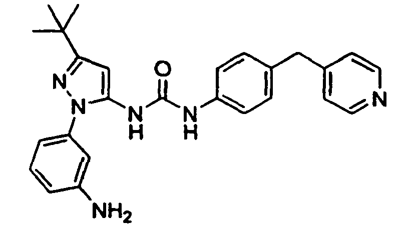

***N*-(1-(3-Aminophenyl)-3-*tert*-butyl-5-pyrazolyl)-*N'*-(4-(4-pyridinylthio)phenyl)urea:** A solution of *N*-(1-(3-nitrophenyl)-3-*tert*-butyl-5-pyrazolyl]-*N'*-(4-(4-pyridinylthio)phenyl)urea (Prepared in methods analogous to those described in A1 and C1a; 0.310 g, 0.635 mmol) in acetic acid (20 mL) was placed under an atmosphere of Ar using a vacuum-degassed and argon-purge protocol. To this was added water (0.2 mL) followed by iron powder (325 mesh; 0.354 g, 6.35 mmol). The reaction mixture was stirred vigorously under argon at room temp. for 18 h, at which time TLC indicated the absence of starting material. The reaction mixture was filtered and the solids were washed copiously with water (300 mL). The orange solution was then brought to pH 4.5 by addition of NaOH pellets (a white precipitate forms). The resulting suspension was extracted with Et₂O (3 x 250 mL), and the combined organic layers were washed with a saturated NaHCO₃ solution (2 x 300 mL) until foaming ceased. The resulting solution was dried (MgSO₄) and concentrated under reduced pressure. The resulting white solid was purified by column chromatography (gradient from 30% acetone/70% CH₂Cl₂ to 50% acetone/50% CH₂Cl₂) to give the product as a white solid (0.165 g, 57%): TLC (50% acetone/50% CH₂Cl₂) R_{*f*}0.50; ¹H NMR (DMSO-d₆) δ 1.24 (s, 9H), 5.40 (br s, 2H), 6.34 (s, 1H), 6.57 (d, *J*=8 Hz, 2H), 6.67 (s, 1H), 6.94 (d, *J*=6 Hz, 2H), 7.12 (app t, *J*=8 Hz, 1H), 7.47 (d, *J*=9 Hz, 2H), 7.57 (d, *J*=9 Hz, 2H), 8.31 (d, *J*=6 Hz, 2H), 8.43 (s, 1H), 9.39 (s, 1H); FAB-MS *m*/*z* 459 ((M+H)⁺).

### D4. General Methods of Acylation of Amine-Containing Aryl Ureas

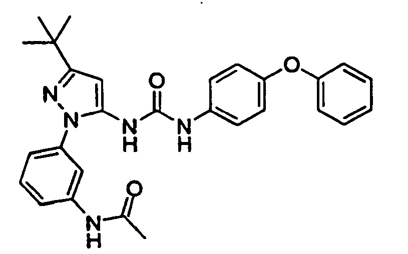

***N*-(1-(3-Acetamidophenyl)-3-*tert*-butyl-5-pyrazolyl)-*N'*-(4-phenoxyphenyl)urea:** To a solution of *N*-(1-(3-aminophenyl)-3-*tert*-butyl-5-pyrazolyl)-*N'*-(4-phenoxyphenyl)urea (prepared using methods analogous to those described in A1, C1a and D3; 0.154 g, 0.349 mmol) in CH₂Cl₂ (10 mL) was added pyridine (0.05 mL) followed by acetyl chloride (0.030 mL, 0.417 mmol). The reaction mixture was stirred under argon at room temp. for 3 h, at which time TLC analysis indicated the absence of starting material. The reaction mixture was diluted with CH₂Cl₂(20 mL), then the resulting solution was sequentially washed with water (30 mL) and a saturated NaCl solution (30 mL), dried (MgSO₄) and concentrated under reduced pressure. The resulting residue was purified by column chromatography (gradient from 5% EtOAc/95% hexane to 75% EtOAc/25% hexane) to give the product as a white solid (0.049 g, 30%): TLC (70% EtOAc/30% hexane) R_{*f*} 0.32; ¹H NMR (DMSO-d₆) δ 1.26 (s, 9H), 2.05 (s, 3H), 6.35 (s, 1H), 6.92-6.97 (m, 4H), 7.05-7.18 (m, 2H), 7.32-7.45 (m, 5H), 7.64-7.73 (m, 2H), 8.38 (s, 1H), 9.00 (s, 1H), 10.16 (s, 1H); FAB-MS *m*/*z* 484 ((M+H)⁺).

The following compounds have been synthesized according to the General Methods listed above:

**Table 1. 2-Substituted-5-tert-butylpyrazolyl Ureas**

| | | | | | | | |
|---|---|---|---|---|---|---|---|
| 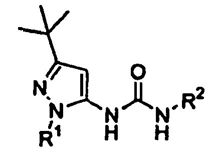 | | | | | | | |

| Entry | R¹ | R² | mp (°C) | TLC R_{f} | Solvent System | Mass Spec. [Source] | Synth. Method |
|---|---|---|---|---|---|---|---|
| 18 | 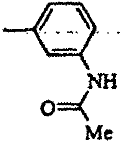 | 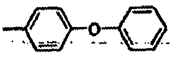 | | 0.31 | 70% EtOAc/ 30% hexane | 484 (M+H)⁺ [FAB] | A1, C1a, D3, D4 |
| 19 | 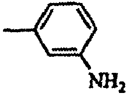 | 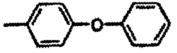 | | 0.14 | 50% EtOAc/ 50% hexane | 442 (M+H)+ [FAB] | A1, C1a, D3 |
| 20 | 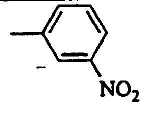 | 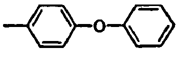 | | 0.19 | 30% EtOAc/ 70% hexane | 472 (M+H)+ [FAB] | Al, C1a |
| 21 | 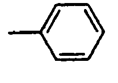 | 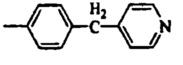 | | 0.56 | 60% acetone /40% CH2Cl 2 | 426 (M+H)+ [FAB] | A1, C2 |
| 22 | 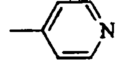 | 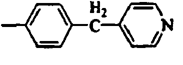 | | 0.34 | 10% MeOH/ 90% CH2Cl 2 | 427 (M+H)+ [FAB] | A1, C2 |
| 23 | 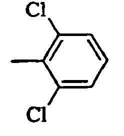 | 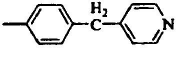 | | 0.44 | 40% acetone /60% CH2Cl 2 | 494 (M+H)+ [FAB] | A1, C2 |
| 24 | 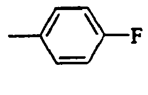 | 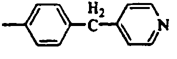 | | 0.44 | 40% acetone / 60% CH2C1 2 | 444 (M+H)+ [FAB] | A1, C2 |
| 25 | 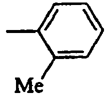 | 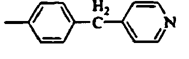 | | 0.46 | 40% acetone /60% CH2Cl 2 | 440 (M+H)+ [FAB] | A1, C2 |
| 26 | 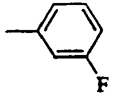 | 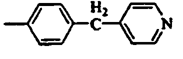 | | 0.48 | 40% acetone /60% CH2Cl 2 | 444 (M+H)+ [FAB] | A1, C2 |
| 27 | 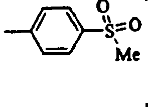 | 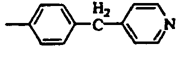 | | 0.34 | 40% acetone / 60% CH2C1 2 | 504 (M+H)+ | A1, C2 |
| 28 | 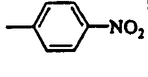 | 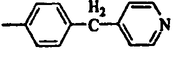 | | 0.47 | 40% acetone / 60% CH2Cl 2 | 471 (M+H)+ [FAB] | A1, C2 |
| 29 | 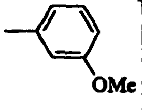 | 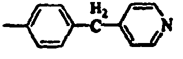 | | 0.51 | 60% acetone /40% CH2Cl 2 | 456 (M+H)+ [FAB] | A1, C2 |
| 30 | 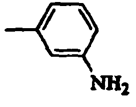 | 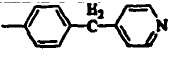 | | 0.50 | 50% acetone /50% CH2Cl 2 | 441 (M+H)+ [FAB] | A1, C2, D3 |
| 31 | 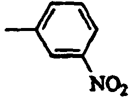 | 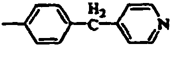 | | 0.43 | 30% acetone /70% CH2Cl 2 | 471 (M+H)+ [FAB] | A1, C2 |
| 32 | 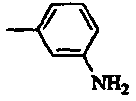 | 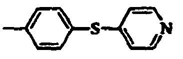 | | 0.50 | 50% acetone /50% CH2Cl 2 | 459 (M+H)+ [FAB] | A1, C2, D3 |
| 33 | 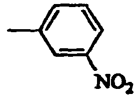 | 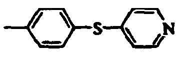 | | 0.47 | 30% acetone /70% CH2C1 2 | 489 (M+H)+ [FAB] | A1, C2 |
| 34 | 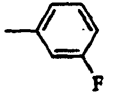 | 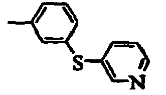 | | | | 461 (M+H)+ [FAB] | A1, C2 |
| 35 | 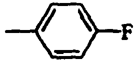 | 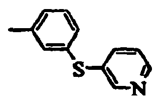 | | | | 461 (M+H)+ [FAB] | A1, C2 |
| 36 | 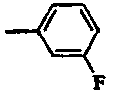 | 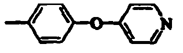 | | | | 445 (M+H)+ [FAB] | A1, C2 |
| 37 | 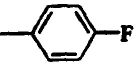 | 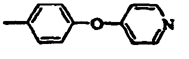 | | | | 445 (M+H)+ [FAB] | A1, C2 |

### BIOLOGICAL EXAMPLES

### P38 Kinase Assay:

The *in vitro* inhibitory properties of compounds were determined using a p38 kinase inhibition assay. P38 activity was detected using an *in vitro* kinase assay run in 96-well microtiter plates. Recombinant human p38 (0.5 µg/mL) was mixed with substrate (myelin basic protein, 5 µg/mL) in kinase buffer (25 mM Hepes, 20 mM MgCl₂ and 150 mM NaCl) and compound. One µCi/well of ³³P-labeled ATP (10 µM) was added to a final volume of 100 µL. The reaction was run at 32 °C for 30 min. and stopped with a 1M HCl solution. The amount of radioactivity incorporated into the substrate was determined by trapping the labeled substrate onto negatively charged glass fiber filter paper using a 1% phosphoric acid solution and read with a scintillation counter. Negative controls include substrate plus ATP alone.

All compounds exemplified displayed p38 IC₅₀s of between 1 nM and 10 µM.

### LPS Induced TNFα Production in Mice:

The *in vivo* inhibitory properties of selected compounds were determined using a murine LPS induced TNFα production *in vivo* model. BALB/c mice (Charles River Breeding Laboratories; Kingston, NY) in groups of ten were treated with either vehicle or compound by the route noted. After one hour, endotoxin (E. coli lipopolysaccharide (LPS) 100 µg) was administered intraperitoneally (i.p.). After 90 min, animals were euthanized by carbon dioxide asphyxiation and plasma was obtained from individual animal by cardiac puncture ionto heparinized tubes. The samples were clarified by centrifugation at 12,500 x g for 5 min at 4 °C. The supernatants were decanted to new tubes, which were stored as needed at -20 °C. TNFα levels in sera were measured using a commercial murine TNF ELISA kit (Genzyme).

The preceeding examples can be repeated with similar success by substituting the generically of specifically described reactants and/or operating conditions of this invention for those used in the preceeding examples.

## Claims

1. Use of a compound of formula I or a pharmaceutically acceptable salt thereof 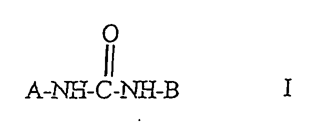 wherein A is a heteroaryl selected from the group consisting of 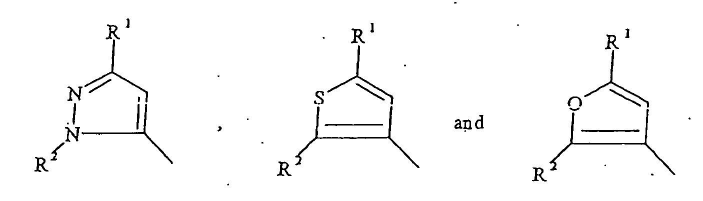 wherein R¹ is selected from the group consisting of C₃-C₁₀ alkyl, C₃-C₁₀ cycloalkyl, up to per-halosubstituted C₁-C₁₀ alkyl and up to per-halosubstituted C₃-C₁₀ cycloalkyl,
wherein B is an up to tricyclic, aryl or heteroaryl moiety of up to 30 carbon atoms with at least one 5- or 6-member aromatic structure containing 0-4 members of the group consisting of nitrogen, oxygen and sulfur
substituted by -Y-Ar; and is optionally substituted by one or more substitutents independently selected from the group consisting of halogen, up to per-halosubstitution, and Xₙ,
wherein n is 0-2 and each X is independently selected from the group consisting of -CN, CO₂R⁵, -C(O)NR⁵R^{5'}, -C(O)R⁵, -NO₂, -OR⁵, -SR⁵, -NR⁵R^{5'}, -NR⁵C(O)OR^{5'}, -NR⁵C(O)R^{5'}, C₁-C₁₀ alkyl, C₂₋₁₀-alkenyl, C₁₋₁₀-alkoxy, C₃-C₁₀ cycloalkyl, C₆-C₁₄ aryl, C₇-C₂₄ alkaryl, C₃-C₁₃ heteroaryl, C₄-C₂₃ alkheteroaryl, substituted C₁-C₁₀ alkyl, substituted C₂₋₁₀-alkenyl, substituted C₁₋₁₀-alkoxy, substituted C₅-C₁₀ cycloalkyl, substituted C₄-C₂₃ alkheteroaryl and -Y-Ar,
where X is a substituted group, it is substituted by one or more substituents independently selected from the group consisting of -CN, -CO₂R⁵, -C(O)R⁵, -C(O)NR⁵R⁵, -OR⁵, -SR⁵, -NR⁵R^{5'}, -NO₂, -NR⁵C(O)R^{5'}, -NR⁵C(O)OR^{5'} and halogen up to per-halosubstitution;
wherein R⁵ and R^{5'} are independently selected from H, C₁-C₁₀ alkyl, C₂₋₁₀-alkenyl, C₃-C₁₀ cycloalkyl, C₆-C₁₄ aryl, C₃-C₁₃ heteroaryl, C₇-C₂₄ alkaryl, C₄-C₂₃ alkheteroaryl, up to per-halosubstituted C₁-C₁₀ alkyl, up to per-halosubstituted C₂₋₁₀-alkenyl, up to per-halosubstituted C₃-C₁₀ cycloalkyl, up to per-halosubstituted C₆-C₁₄ aryl and up to per-halosubstituted C₃-C₁₃ heteroaryl,
wherein Y is selected from the group consisting of -O-, -S-, -N(R⁵)-, -(CH₂)-ₘ, -C(O)-, -CH(OH)-, -(CH₂)ₘO-, -NR⁵C(O)NR⁵R^{5'}-, -NR⁵C(O)-, -C(O)NR⁵-, -(CH₂)ₘS-, -(CH₂)ₘN(R⁵)-, -O(CH₂)ₘ-, -CHX^{a}-, -CX^{a}₂-, -S-(CH₂)ₘ- and -N(R⁵)(CH₂)ₘ-,
m = 1-3, and X^{a} is halogen; and
Ar is a 5-10 member aromatic structure containing 0-2 members of the group consisting of nitrogen, oxygen and sulfur which is unsubstituted or substituted by halogen up to per-halosubstitution and optionally substituted by Zₘ₁, wherein n1 is 0 to 3 and each Z is independently selected from the group consisting of -CN, -CO₂R⁵, -C(O)NR⁵R^{5'}, -C(O)NR⁵H,-NO₂, -OR⁵, -SR⁵, -NR⁵R^{5'}, -NR⁵C(O)OR^{5'}, -OC(O)R⁵, -NR⁵C(O)R^{5'}, C₁-C₁₀ alkyl, C₃-C₁₀ cycloalkyl, C₆-C₁₄ aryl, C₃-C₁₃ heteroaryl, C₇-C₂₄ alkaryl, C₄-C₂₃ alkheteroaryl, substituted C₁-C₁₀ alkyl, substituted C₃-C₁₀ cycloalkyl, substituted C₇-C₂₄ alkaryl and substituted C₄-C₂₃ alkheteroaryl;
wherein if Z is a substituted group, it is substituted by the one or more substituents independently selected from the group consisting of -CN, -CO₂R⁵, -C(O)NR⁵R^{5'}, -OR⁵, -SR⁵, -NO₂, -NR⁵R⁵, -NR⁵C(O)R^{5'} and -NR⁵C(O)OR^{5'}, and
wherein R² is C₆-C₁₄ aryl, C₃-C₁₄ heteroaryl, substituted C₆-C₁₄ aryl or substituted C₃-C₁₄ heteroaryl ,
wherein if R² is a substituted group, it is substituted by one or more substituents independently selected from the group consisting of halogen, up to per-halosubstitution, and Vₙ,
wherein n = 0-3 and each V is independently selected from the group consisting of -CN, -CO₂R⁵, -C(O)NR⁵R^{5'}, -OR⁵, -SR⁵, -NR⁵R^{5'}, -C(O)R⁵, -OC(O)NR⁵R^{5'}, -NR⁵C(O)OR^{5'}, -SO₂R⁵, -SOR⁵, -NR⁵C(O)R^{5'}, -NO₂ C₁-C₁₀ alkyl, C₃-C₁₀ cycloalkyl, C₆-C₁₄ aryl, C₃-C₁₃ heteroaryl, C₇-C₂₄ alkaryl, C₄-C₂₄ alkheteroaryl, substituted C₁-C₁₀ alkyl, substituted C₃-C₁₀ cycloalkyl, substituted C₆-C₁₄ aryl, substituted C₃-C₁₃ heteroaryl, substituted C₇-C₂₄ alkaryl and substituted C₄-C₂₄ alkheteroaryl,
where V is a substituted group, it is substituted by one or more substituents independently selected from the group consisting of halogen, up to per-halosubstitution, -CN*,* -CO₂R⁵, -C(O)R⁵, -C(O)NR⁵R⁵, -NR⁵R^{5'}, -OR⁵, -SR⁵, -NR⁵C(O)R⁵, -NR⁵C(O)OR⁵ and -NO₂,
wherein R⁵ and R^{5'} are each independently as defined above, for the preparation of a medicament for the treatment of acute and chronic inflammatory and/or immunomodulatory diseases other than cancer.

2. Use as in claim 1 wherein R² is phenyl, substituted phenyl, pyridinyl or substituted pyridinyl.

3. Use as in claim 1, wherein B is an aromatic ring structure selected from the group consisting of 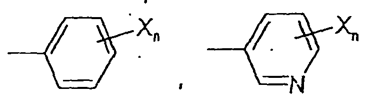 which is substituted or unsubstituted by halogen, up to per-halosubstitution, and wherein
n=1-3 and
at least one X is -Y-Ar, and
any additional X is independently selected from the group consisting of -CN, -CO₂R⁵, -C(O)NR⁵R^{5'}, -C(O)R⁵, -NO₂, -OR⁵, - SR⁵, - NR⁵R^{5'}, -NR⁵C(O)OR^{5'}, -NR⁵C(O)R^{5'}, C₁-C₁₀ alkyl, C₃-C₁₀ cycloalkyl, C₆-C₁₄ aryl, C₇-C₂₄ alkaryl, C₃-C₁₃ heteroaryl, C₄-C₂₃ alkheteroaryl, and substituted C₁-C₁₀ alkyl, substituted C₃-C₁₀ cycloalkyl, substituted C₄-C₂₃ alkheteroaryl and -Y-Ar;
wherein if X is a substituted group, it is substituted by one or more substituents independently selected from the group consisting of -CN, -CO₂R⁵, -C(O)R⁵, -C(O)NR⁵R^{5'}, -OR⁵, -SR⁵, -NR⁵R^{5'}, -NO₂, -NR⁵C(O)R^{5'}, -NR⁵C(O)OR^{5'} and halogen up to per-halosubstitution;
wherein R⁵ and R^{5'} are independently selected from H, C₁-C₁₀ alkyl, C₅-C₁₀ cycloalkyl, C₆-C₁₄ aryl, C₃-C₁₃ heteroaryl, C₇-C₂₄ alkaryl, C₄-C₂₃ alkheteroaryl, up to per-halosubstituted C₁-C₁₀ alkyl, up to per-halosubstituted C₃-C₁₀ cycloalkyl, up to per-halosubstituted C₆-C₁₄ aryl and up to per-halosubstituted C₃-C₁₃ heteroaryl,
wherein Y is selected from the group consisting of -O-, -S-, -N(R⁵)-, -(CH₂)-ₘ, -C(O)-, -CH(OH)-, -(CH₂)ₘO-, -(CH₂)ₘS-, -(CH₂)ₘN(R⁵)-, -O(CH₂)ₘ-, -CHX^{a}-, -CX^{a}₂-, -S-(CH₂)ₘ- and -N-(R⁵)(CH₂)ₘ-,
m = 1-3, and X^{a} is halogen; and
Ar is a 5- or 6-member aromatic structure containing 0-2 members of the group consisting of nitrogen, oxygen and sulfur which is unsubstituted or substituted by halogen up to per-halosubsntution and optionally substituted by Zₙ₁, wherein n1 is 0 to 3 and each Z is independently selected from the group consisting of -CN, -CO₂R⁵, -C(O)NR⁵R^{5'}, -C(O)R⁵, -NO₂, -OR⁵, - SR⁵, - NR⁵R^{5'}, -NR⁵C(O)OR^{5'}, -NR⁵C(O)R^{5'}, C₁-C₁₀ alkyl, C₃-C₁₀ cycloalkyl, C₆-C₁₄ aryl, C₃-C₁₃ heteroaryl, C₇-C₂₄ alkaryl, C₄-C₂₃ alkheteroaryl, substituted C₁-C₁₀ alkyl, substituted C₃-C₁₀ cycloalkyl, substituted C₇-C₂₄ alkaryl and substituted C₄-C₂₃ alkheteroaryl; wherein if Z is a substituted group, it is substituted by one or more substituents independently selected from the group consisting of -CN, -CO₂R⁵, -C(O)NR⁵R^{5'}, -OR⁵, -SR⁵, -NO₂, -NR⁵R^{5'}, -NR⁵C(O)R^{5'} and -NR⁵C(O)OR^{5'}.

4. Use as in claim 1, wherein Y is selected from the group consisting of -S-, -CH₂-, -SCH₂-, -CH₂S-, -CH(OH)-, -C(O)-, -CX^{a}₂, -CX^{a}H-, -CH₂O- and -OCH₂-, and X^{a} is halogen.

5. Use as in claim 1, wherein Y is -0- or -CH₂-,

6. Use as in claim 1, wherein Ar is phenyl or pyridinyl.

7. Use as in claim 4, wherein B is phenyl and Ar is phenyl or pyridinyl.

8. Use as in claim 5, wherein B is phenyl and Ar is phenyl or pyridinyl.

9. Use as in claim 6, wherein B is phenyl.

10. Use as in claim 1, wherein the compound is one of the formulae or a pharmaceutically acceptable salt thereof: 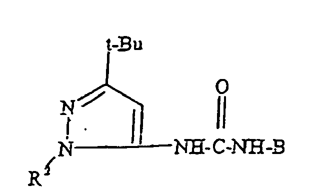 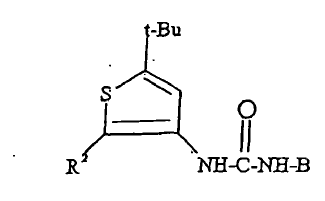 or 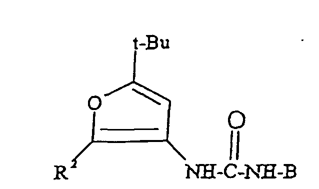 wherein B and R² are as defined in claim 1.

11. Use as in claim 3, wherein R² is selected from substituted and unsubstituted members of the group consisting of phenyl and pyridinyl, wherein if R² is a substituted group, it is substituted by one or more substituents selected from the group consisting of halogen and Wₙ, wherein n = 0-3, and W is selected from the group consisting of -NO₂, -C₁₋₃ alkyl, -NH(O)CH₃, -CF₃, -OCH₃, -F, -Cl, -NH₂, -SO₂CH₃, pyridinyl, phenyl, up to per-halosubstituted phenyl and C₁-C₆ alkyl substituted phenyl.

12. Use as in claim 1, wherein the compound of formula I displays p38 activity (IC₅₀) better than 10µM as determined by an in-vitro kinase assay.

13. Use according to claim 1, wherein the disease is rheumatoid arthritis, osteoarthritis, septic arthritis; osteoporosis, postmenopausal osteoporosis, asthma, septic shock, inflammatory bowel disease or the result of host-versus-graft reactions.

## Patentansprüche

1. Verwendung einer Verbindung der Formel I oder eines pharmazeutisch akzeptablen Salzes davon 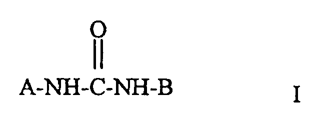 wobei A ein Heteroaryl ausgewählt aus der Gruppe bestehend aus 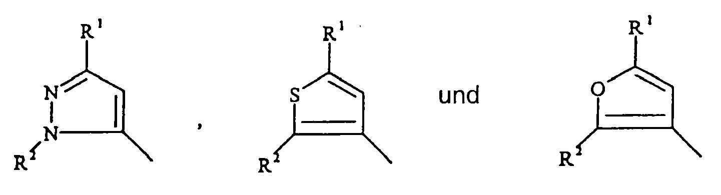 ist,
wobei R₁ ausgewählt ist aus der Gruppe bestehend aus C₃-C₁₀-Alkyl, C₃-C₁₀-Cycloalkyl, bis zu perhalosubstituiertem C₁-C₁₀-Alkyl und bis zu perhalosubstituiertem C₃-C₁₀-Cycloalkyl;
wobei B eine bis zu tricyclische Aryl- oder Heteroarylgruppe von bis zu 30 Kohlenstoffatomen mit wenigstens einer 5- oder 6-gliedrigen aromatischen Struktur ist, die 0-4 Mitglieder aus der Gruppe bestehend aus Stickstoff, Sauerstoff und Schwefel enthält, welche durch -Y-Ar substituiert ist und gegebenenfalls durch einen oder mehrere Substituenten substituiert ist, die unabhängig ausgewählt sind aus der Gruppe bestehend aus Halogen bis zur Perhalosubstitution und Xₙ,
wobei n 0-2 ist und jedes X unabhängig ausgewählt ist aus der Gruppe bestehend aus -CN, CO₂R⁵, -C(O)NR⁵R^{5'}, -C(O)R⁵, -NO₂, -OR⁵, -SR⁵, -NR⁵R^{5'}, -NR⁵C(O)OR^{5'} -NR⁵C(O)R^{5'}, C₁-C₁₀-Alkyl, C₂₋₁₀-Alkenyl, C₁₋₁₀-Alkoxy, C₃-C₁₀-Cycloalkyl, C₆-C₁₄-Aryl, C₇-C₂₄-Alkaryl, C₃-C₁₃-Heteroaryl, C₄-C₂₃-Alkheteroaryl, substituiertem C₁-C₁₀-Alkyl, substituiertem C₂₋₁₀-Alkenyl, substituiertem C₁₋₁₀-Alkoxy, substituiertem C₃-C₁₀-Cycloalkyl, substituiertem C₄-C₂₃-Alkheteroaryl und -Y-Ar;
wobei wenn X eine substituierte Gruppe ist, ist es durch einen oder mehrere Substituenten substituiert unabhängig ausgewählt aus der Gruppe bestehend aus -CN, -CO₂R⁵, -C(O)R⁵, -C(O)NR⁵R^{5'}, -OR⁵, -SR⁵, -NR⁵R^{5'}, -NO₂, -NR⁵C(O)R^{5'}, -NR⁵C(O)OR^{5'} und Halogen bis zur Perhalosubstitution;
wobei R⁵ und R^{5'} unabhängig ausgewählt sind aus H, C₁-C₁₀-Alkyl, C₂₋₁₀-Alkenyl, C₃-C₁₀-Cycloalkyl, C₆-C₁₄-Aryl, C₃-C₁₃-Heteroaryl, C₇-C₂₄-Alkaryl, C₄-C₂₃-Alkheteroaryl, bis zu perhalosubstituiertem C₁-C₁₀-Alkyl, bis zu perhalosubstituiertem C₂-₁₀-Alkenyl, bis zu perhalosubstituiertem C₃-C₁₀-Cycloalkyl, bis zu perhalosubstituiertem C₆-C₁₄-Aryl und bis zu perhalosubstituiertem C₃-C₁₃-Heteroaryl,
wobei Y ausgewählt ist aus der Gruppe bestehend aus -O-, -S-, -N(R⁵)-, -(CH₂)-ₘ, -C(O)-, -CH(OH)-, -(CH₂)ₘO-, -NR⁵C(O)NR⁵R^{5'}-, -NR⁵C(O)-, -C(O)NR⁵-, -(CH₂)ₘS-, -(CH₂)ₘN(R⁵)-, -O(CH₂)ₘ-, -CHX^{a}-, -CX^{a}₂-, -S-(CH₂)ₘ-und -N(R⁵)(CH₂)ₘ-,
m = 1-3 ist und X^{a} Halogen ist; und
Ar eine 5-10-gliedrige aromatische Struktur ist, die 0-2 Mitglieder aus der Gruppe bestehend aus Stickstoff, Sauerstoff und Schwefel enthält, die unsubstituiert oder substituiert ist durch Halogen bis zur Perhalosubstitution und gegebenenfalls substituiert ist durch Zₙ₁, wobei n1 0 bis 3 ist und jedes Z unabhängig ausgewählt ist aus der Gruppe bestehend aus -CN, -C0₂R⁵, -C(O)NR⁵R^{5'}, -C(O)NR⁵H, -NO₂, -OR⁵, -SR⁵, -NR⁵R^{5'}, -NR⁵C(O)OR^{5'}, -OC(O)R⁵, -NR⁵C(O)R^{5'}, C₁-C₁₀-Alkyl, C₃-C₁₀-Cycloalkyl, C₆-C₁₄-Aryl, C₃-C₁₃-Heteroaryl, C₇-C₂₄-Alkaryl, C₄-C₂₃-Alkheteroaryl, substituiertem C₁-C₁₀-Alkyl, substituiertem C₃-C₁₀-Cycloalkyl, substituiertem C₇-C₂₄-Alkaryl und substituiertem C₄-C₂₃-Alkheteroaryl;
wobei wenn Z eine substituierte Gruppe ist, ist es durch einen oder mehrere Substituenten substituiert unabhängig ausgewählt aus der Gruppe bestehend aus -CN, -CO₂R⁵, -C(O)NR⁵R^{5'}, -OR⁵, -SR⁵, -NO₂, -NR⁵R^{5'}, -NR⁵C(O)R^{5'} und -NR⁵C(O)OR^{5'}, und
wobei R² C₆-C₁₄-Aryl, C₃-C₁₄-Heteroaryl, substituiertes C₆-C₁₄-Aryl oder substituiertes C₃-C₁₄-Heteroaryl ist,
wobei wenn R² eine substituierte Gruppe ist, ist sie substituiert durch einen oder mehrere Substituenten unabhängig ausgewählt aus der Gruppe bestehend aus Halogen bis zur Perhalosubstitution und Vₙ,
wobei n = 0-3 ist und jedes V unabhängig ausgewählt ist aus der Gruppe bestehend aus -CN, -CO₂R⁵, -C(O)NR⁵R^{5'}, -OR⁵, -SR⁵, -NR⁵R^{5'}, -C(O)R⁵, -OC(O)NR⁵R^{5'}, -NR⁵C(O)OR^{5'}, -SO₂R⁵, -SOR⁵, -NR⁵C(O)R^{5'}, -NO₂, C₁-C₁₀-Alkyl, C₃-C₁₀-Cycloalkyl, C₆-C₁₄-Aryl, C₃-C₁₃-Heteroaryl, C₇-C₂₄-Alkaryl, C₄-C₂₄-Alkheteroaryl, substituiertem C₁-C₁₀-Alkyl, substituiertem C₃-C₁₀-Cycloalkyl, substituiertem C₆-C₁₄-Aryl, substituiertem C₃-C₁₃-Heteroaryl, substituiertem C₇-C₂₄-Alkaryl und substituiertem C₄-C₂₄-Alkheteroaryl,
wobei wenn V eine substituierte Gruppe ist, ist sie substituiert durch einen oder mehrere Substituenten unabhängig ausgewählt aus der Gruppe bestehend aus Halogen bis zur Perhalosubstitution, -CN, -CO₂R⁵, -C(O)R⁵, -C(O)NR⁵R⁵, -NR⁵R^{5'}, -OR⁵, -SR⁵, -NR⁵C(O)R^{5'}, -NR⁵C(O)OR^{5'} und -NO₂;
wobei R⁵ und R^{5'} jeweils unabhängig wie oben definiert sind,
zur Herstellung eines Medikamentes zur Behandlung von akuten und chronischen entzündlichen und/oder immunmodulatorischen Erkrankungen außer Krebs.

2. Verfahren nach Anspruch 1, wobei R² Phenyl, substituiertes Phenyl, Pyridinyl oder substituiertes Pyridinyl ist.

3. Verfahren nach Anspruch 1, wobei B eine aromatische Ringstruktur ist ausgewählt aus der Gruppe bestehend aus 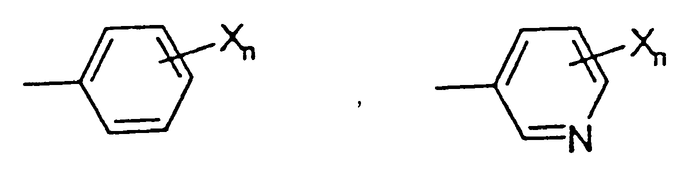 welche substituiert oder unsubstituiert ist durch Halogen bis zur Perhalosubstitution, und wobei
n = 1-3 ist, und
wenigstens ein X -Y-Ar ist, und
jedes zusätzliche X unabhängig ausgewählt ist aus der Gruppe bestehend aus -CN, -CO₂R⁵, -C(O)NR⁵R^{5'}, -C(O)R⁵, -NO₂, -OR⁵, -SR⁵, -NR⁵R^{5'}, -NR⁵C(O)OR^{5'}, -NR⁵C(O)R^{5'}, C₁-C₁₀-Alkyl, C₃-C₁₀-Cycloalkyl, C₆-C₁₄-Aryl, C₇-C₂₄-Alkaryl, C₃-C₁₃-Heteroaryl, C₄-C₂₃-Alkheteroaryl und substituiertem C₁-C₁₀-Alkyl, substituiertem C₃-C₁₀-Cycloalkyl, substituiertem C₄-C₂₃-Alkheteroaryl und -Y-Ar;
wobei wenn X eine substituierte Gruppe ist, ist sie substituiert durch einen oder mehrere Substituenten unabhängig ausgewählt aus der Gruppe bestehend aus -CN, -CO₂R⁵, -C(O)R⁵, -C(O)NR⁵R^{5'}, -OR⁵, -SR⁵, -NR⁵R^{5'}, -NO₂, -NR⁵C(O)R^{5'}, -NR⁵C(O)OR^{5'} und Halogen bis zur Perhalosubstitution;
wobei R⁵ und R^{5'} unabhängig ausgewählt sind aus H, C₁-C₁₀-Alkyl, C₃-C₁₀-Cycloalkyl, C₆-C₁₄-Aryl, C₃-C₁₃-Heteroaryl, C₇-C₂₄-Alkaryl, C₄-C₂₃-Alkheteroaryl, bis zu perhalosubstituiertem C₁-C₁₀-Alkyl, bis zu perhalosubstituiertem C₃-C₁₀-Cycloalkyl, bis zu perhalosubstituierem C₆-C₁₄-Aryl und bis zu perhalosubstituiertem C₃-C₁₃-Heteroaryl,
wobei Y aus der Gruppe ausgewählt ist bestehend aus -O-, -S-, -N(R⁵)-, -(CH₂)-ₘ, -C(O)-, -CH(OH)-, -(CH₂)ₘO-, -(CH₂)ₘS-, -(CH₂)ₘN(R⁵)-, -O(CH₂)ₘ-, -CHX^{a}-, -CX^{a}₂-, -S-(CH₂)ₘ- und -N(R⁵)(CH₂)ₘ-,
m = 1-3 und X^{a} Halogen ist; und
Ar eine 5- oder 6-gliedrige aromatische Struktur ist, die 0-2 Mitglieder aus der Gruppe bestehend aus Stickstoff, Sauerstoff und Schwefel enthält, die unsubstituiert oder substituiert ist durch Halogen bis zur Perhalosubstitution und gegebenenfalls substituiert durch Zₙ₁ ist, wobei n1 0 bis 3 ist und jedes Z unabhängig ausgewählt ist aus der Gruppe bestehend aus -CN, -CO₂R⁵, -C(O)NR⁵R^{5'}, -C(O)R⁵, -NO₂, -OR⁵, -SR⁵, -NR⁵R^{5'}, -NR⁵C(O)OR^{5'}, -NR⁵C(O)R^{5'}, C₁-C₁₀-Alkyl, C₃-C₁₀-Cycloalkyl, C₆-C₁₄-Aryl, C₃-C₁₃-Heteroaryl, C₇-C₂₄-Alkaryl, C₄-C₂₃-Alkheteroaryl, substituiertem C₁-C₁₀-Alkyl, substituiertem C₃-C₁₀-Cycloalkyl, substituiertem C₇-C₂₄-Alkaryl und substituiertem C₄-C₂₃-Alkheteroaryl, wobei wenn Z eine substituierte Gruppe ist, ist sie substituiert durch einen oder mehrere Substituenten unabhängig ausgewählt aus der Gruppe bestehend aus -CN, -CO₂R⁵, -C(O)NR⁵R^{5'}, -OR⁵, -SR⁵, -NO₂, -NR⁵R^{5'}, -NR⁵C(O)R^{5'}, -NR⁵C(O)OR^{5'}.

4. Verwendung nach Anspruch 1, wobei Y ausgewählt ist aus der Gruppe bestehend aus -S-, -CH₂-, -SCH₂-, -CH₂S-, -CH(OH)-, -C(O)-, -CX^{a}₂, -CX^{a}H-, -CH₂O- und -OCH₂-, und X^{a} Halogen ist.

5. Verwendung nach Anspruch 1, wobei Y -O- oder -CH₂- ist.

6. Verwendung nach Anspruch 1, wobei Ar Phenyl oder Pyridinyl ist.

7. Verwendung nach Anspruch 4, wobei B Phenyl ist und Ar Phenyl oder Pyridinyl ist.

8. Verwendung nach Anspruch 5, wobei B Phenyl ist und Ar Phenyl oder Pyridinyl ist.

9. Verwendung nach Anspruch 6, wobei B Phenyl ist.

10. Verwendung nach Anspruch 1, wobei die Verbindung eine Verbindung der Formeln oder ein pharmazeutisch akzeptables Salz davon ist: 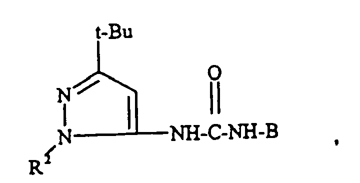 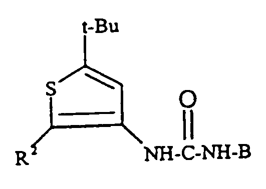 oder 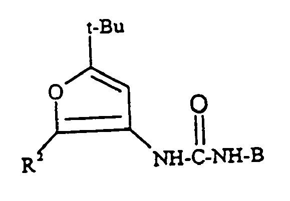 wobei B und R² wie in Anspruch 1 definiert sind.

11. Verwendung nach Anspruch 3, wobei R² ausgewählt ist aus substituierten oder unsubstituierten Mitgliedern der Gruppe bestehend aus Phenyl und Pyridinyl, wobei wenn R² eine substituierte Gruppe ist, ist sie substituiert durch einen oder mehrere Substituenten ausgewählt aus der Gruppe bestehend aus Halogen und Wₙ, wobei n = 0-3 ist und W ausgewählt ist aus der Gruppe bestehend aus -NO₂, C₁₋₃-Alkyl, -NH(O)CH₃, -CF₃, -OCH₃, -F, -CI, -NH₂, -SO₂CH₃, Pyridinyl, Phenyl, bis zu perhalosubstituiertem Phenyl und C₁-C₆-alkylsubstiutiertem Phenyl.

12. Verwendung nach Anspruch 1, wobei die Verbindung der Formel I eine p38-Aktivitat (IC₅₀) von besser als 10 µM zeigt, wie durch einen in-vitro-Kinasetest bestimmt wird.

13. Verwendung nach Anspruch 1, wobei die Krankheit rheumatoide Arthritis, Osteoarthritis, septische Arthritis, Osteoporose, postmenopausale Osteoporose, Asthma, septischer Schock, inflammatorische Darmerkrankung, oder das Ergebnis von Wirt-gegen-Transplantat-Reaktionen ist.

## Revendications

1. Utilisation d'un composé de formule I ou d'un de ses sels pharmaceutiquement acceptables 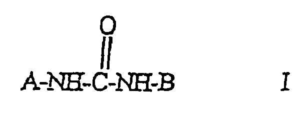 dans laquelle A est un hétéroaryle choisi dans le groupe constitué par 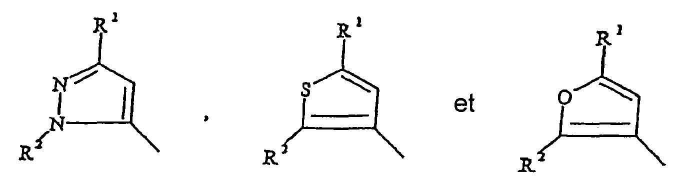 où R¹ est choisi dans le groupe constitué par alkyle en C₃-C₁₀, cycloalkyle en C₃-C₁₀, alkyle en C₁-C₁₀ jusqu'à perhalogéné et cycloalkyle en C₃-C₁₀ jusqu'à perhalogéné,
B est un groupe aryle ou hétéroaryle ayant jusqu'à 3 cycles et jusqu'à 30 atomes de carbone, avec au moins une structure aromatique de 5 ou 6 chaînons contenant 0-4 membres du groupe constitué par l'azote, l'oxygène et le soufre, substitué par -Y-Ar, et éventuellement substitué par un ou plusieurs substituants choisis indépendamment dans le groupe constitué par halogène, jusqu'à la perhalogénation, et Xₙ, où n est 0-2, et chaque X est choisi indépendamment dans le groupe constitué par -CN, CO₂R⁵, -C(O)NR⁵R^{5'}, -C(O)R⁵, -NO₂, -OR⁵, -SR⁵, -NR⁵R^{5'}, -NR⁵C(O)OR^{5'}, -NR⁵C(O)R^{5'}, alkyle en C₁-C₁₀, alcényle en C₂-C₁₀, alcoxy en C₁-C₁₀, cycloalkyle en C₃-C₁₀, aryle en C₆-C₁₄, alkylaryle en C₇-C₂₄, hétéroaryle en C₃-C₁₃, alkylhétéroaryle en C₄-C₂₃, alkyle en C₁-C₁₀ substitué, alcényle en C₂-C₁₀ substitué, alcoxy en C₁-C₁₀ substitué, cycloalkyle en C₃-C₁₀ substitué, alkylhétéroaryle en C₄-C₂₃ substitué et Y-Ar;
lorsque X est un groupe substitué, il est substitué par un ou plusieurs substituants choisis indépendamment dans le groupe constitué par -CN, -CO₂R⁵, -C(O)R⁵, -C(O)NR⁵R^{5'}, -OR⁵, -SR⁵, -NR⁵R^{5'}, -NO₂, -NR⁵C(O)R^{5'}, -NR⁵C(O)OR^{5'} et halogène jusqu'à la perhalogénation;
R⁵ et R^{5'} sont choisis indépendamment parmi H, alkyle en C₁-C₁₀, alcényle en C₂-C₁₀, cycloalkyle en C₃-C₁₀, aryle en C₆-C₁₄, hétéroaryle en C₃-C₁₃, alkylaryle en C₇-C₂₄, alkylhétéroaryle en C₄-C₂₃, alkyle en C₁-C₁₀ jusqu'à perhalogéné, alcényle en C₂-C₁₀ jusqu'à perhalogéné, cycloalkyle en C₃-C₁₀ jusqu'à perhalogéné, aryle en C₆-C₁₄ jusqu'à perhalogéné et hétéroaryle en C₃-C₁₃ jusqu'à perhalogéné,
Y est choisi dans le groupe constitué par -O-, -S-, -N(R⁵)-, -(CH₂)ₘ -, -C(O)-, -CH(OH)-, -(CH₂)ₘO-, -NR⁵C(O)NR⁵R^{5'}-, -NR⁵C(O)-, -C(O)NR⁵-, -(CH₂)ₘS-, -(CH₂)ₘN(R⁵)-, -O(CH₂)ₘ-, -CHX^{a}-, -CX^{a}₂-, -S-(CH₂)ₘ- et -NR⁵(CH₂)ₘ-,
m = 1-3, et X^{a} est un halogène; et
Ar est une structure aromatique de 5-10 chaînons contenant 0-2 membres du groupe constitué par l'azote, l'oxygène et le soufre, qui est non substituée ou substituée par halogène jusqu'à la perhalogénation, et éventuellement substituée par Zₙ₁, où n1 est 0 à 3 et chaque Z est choisi indépendamment dans le groupe constitué par -CN, CO₂R⁵, -C(O)NR⁵R^{5'}, -C(O)NR⁵H, -NO₂, -OR⁵, -SR⁵, -NR⁵R^{5'}, -NR⁵C(O)OR^{5'}, -OC(O)R⁵, -NR⁵C(O)R^{5'}, alkyle en C₁-C₁₀, cycloalkyle en C₃-C₁₀, aryle en C₆-C₁₄, hétéroaryle en C₃-C₁₃, alkylaryle en C₇-C₂₄, alkylhétéroaryle en C₄-C₂₃, alkyle en C₁-C₁₀ substitué, cycloalkyle en C₃-C₁₀ substitué, alkylaryle en C₇-C₂₄ substitué et alkylhétéroaryle en C₄-C₂₃ substitué;
lorsque Z est un groupe substitué, il est substitué par un ou plusieurs substituants choisis indépendamment dans le groupe constitué par -CN, -CO₂R⁵, -C(O)NR⁵R^{5'}, -OR⁵, -SR⁵, -NO₂, -NR⁵R^{5'}, -NR⁵C(O)R^{5'} et -NR⁵C(O)OR^{5'}; et
R² est aryle en C₆-C₁₄, , hétéroaryle en C₃-C₁₄, aryle en C₆-C₁₄ substitué ou hétéroaryle en C₃-C₁₄ substitué,
si R² est un groupe substitué, il est substitué par un ou plusieurs substituants choisis indépendamment dans le groupe constitué par halogène jusqu'à la perhalogénation, et Vₙ,
où n = 0-3 et chaque V est choisi indépendamment dans le groupe constitué par -CN, CO₂R⁵, -C(O)NR⁵R^{5'}, -OR⁵, -SR⁵, -NR⁵R^{5'}, -C(O)R⁵, -OC(O)NR⁵R^{5'}, -NR⁵C(O)OR^{5'}, -SO₂R⁵, -SOR⁵, -NR⁵C(O)R^{5'}, -NO₂, alkyle en C₁-C₁₀, cycloalkyle en C₃-C₁₀, aryle en C₆-C₁₄, hétéroaryle en C₃-C₁₃, alkylaryle en C₇-C₂₄, alkylhétéroaryle en C₄-C₂₄, alkyle en C₁-C₁₀ substitué, cycloalkyle en C₃-C₁₀ substitué, aryle en C₆-C₁₄ substitué, hétéroaryle en C₃-C₁₃ substitué, alkylaryle en C₇-C₂₄ substitué et alkylhétéroaryle en C₄-C₂₄ substitué;
lorsque V est un groupe substitué, il est substitué par un ou plusieurs substituants choisis indépendamment dans le groupe constitué par halogène, jusqu'à la perhalogénation -CN, -CO₂R⁵, -C(O)R⁵, -C(O)NR⁵R^{5'}, -NR⁵R^{5'}, -OR⁵, -SR⁵, -NR⁵C(O)R^{5'}, -NR⁵C(O)OR^{5'} et -NO₂,
où R⁵ et R^{5'} sont chacun indépendamment tels que définis ci-dessus, pour la préparation d'un médicament destiné au traitement de maladies inflammatoires aiguës et chroniques et/ou de maladies immunomodulatrices autres que le cancer.

2. Utilisation selon la revendication 1, dans laquelle R² est phényle, phényle substitué, pyridinyle ou pyridinyle substitué.

3. Utilisation selon la revendication 1, dans laquelle B est une structure cyclique aromatique choisie dans le groupe constitué par 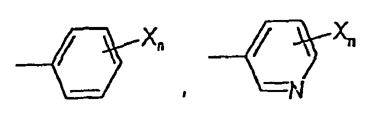 qui est substituée ou non substituée par halogène, jusqu'à la perhalogénation, et dans laquelle
n = 1-3, et
au moins un X est -Y-Ar, et
tout X supplémentaire est choisi indépendamment dans le groupe constitué par -CN, CO₂R⁵, -C(O)NR⁵R^{5'}, -C(O)R⁵, -NO₂, -OR⁵, -SR⁵, -NR⁵R^{5'}, -NR⁵C(O)OR^{5'}, -NR⁵C(O)R^{5'}, alkyle en C₁-C₁₀, cycloalkyle en C₃-C₁₀, aryle en C₆-C₁₄, alkylaryle en C₇-C₂₄, hétéroaryle en C₃-C₁₃, alkylhétéroaryle en C₄-C₂₃, et alkyle en C₁-C₁₀ substitué, cycloalkyle en C₃-C₁₀ substitué, alkylhétéroaryle en C₄-C₂₃ substitué et Y-Ar;
lorsque X est un groupe substitué, il est substitué par un ou plusieurs substituants choisis indépendamment dans le groupe constitué par -CN, -CO₂R⁵, -C(O)R⁵, -C(O)NR⁵R^{5'}, -OR⁵, -SR⁵, -NR⁵R^{5'}, -NO₂, -NR⁵C(O)R^{5'}, -NR⁵C(O)OR^{5'} et halogène jusqu'à la perhalogénation;
R⁵ et R^{5'} sont choisis indépendamment parmi H, alkyle en C₁-C₁₀, cycloalkyle en C₃-C₁₀, aryle en C₆-C₁₄, hétéroaryle en C₃-C₁₃, alkylaryle en C₇-C₂₄, alkylhétéroaryle en C₄-C₂₃, alkyle en C₁-C₁₀ jusqu'à perhalogéné, cycloalkyle en C₃-C₁₀ jusqu'à perhalogéné, aryle en C₆-C₁₄ jusqu'à perhalogéné et hétéroaryle en C₃-C₁₃ jusqu'à perhalogéné,
Y est choisi dans le groupe constitué par -O-, -S-, -N(R⁵)-, -(CH₂)ₘ -, -C(O)-, -CH(OH)-, -(CH₂)ₘO-, -(CH₂)ₘS-, -(CH₂)ₘN(R⁵)-, -O(CH₂)ₘ-, -CHX^{a}-, -CX^{a}₂-, -S-(CH₂)ₘ- et -N(R⁵)(CH₂)ₘ-,
m = 1-3, et X^{a} est un halogène; et
Ar est une structure aromatique de 5 ou 6 chaînons contenant 0-2 membres du groupe constitué par l'azote, l'oxygène et le soufre, qui est non substituée ou substituée par halogène jusqu'à la perhalogénation, et éventuellement substituée par Zₙ₁, où n1 est 0 à 3 et chaque Z est choisi indépendamment dans le groupe constitué par -CN, CO₂R⁵, -C(O)NR⁵R^{5'}, -C(O)R⁵, -NO₂, -OR⁵, -SR⁵, -NR⁵R^{5'}, -NR⁵C(O)OR^{5'}, -NR⁵C(O)R^{5'}, alkyle en C₁-C₁₀, cycloalkyle en C₃-C₁₀, aryle en C₆-C₁₄, hétéroaryle en C₃-C₁₃, alkylaryle en C₇-C₂₄, alkylhétéroaryle en C₄-C₂₃, alkyle en C₁-C₁₀ substitué, cycloalkyle en C₃-C₁₀ substitué, alkylaryle en C₇-C₂₄ substitué et alkylhétéroaryle en C₄-C₂₃ substitué;
lorsque Z est un groupe substitué, il est substitué par un ou plusieurs substituants choisis indépendamment dans le groupe constitué par -CN, -CO₂R⁵, -C(O)NR⁵R^{5'}, -OR⁵, -SR⁵, -NO₂, -NR⁵R^{5'}, -NR⁵C(O)R^{5'} et -NR⁵C(O)OR^{5'}.

4. Utilisation selon la revendication 1, dans laquelle Y est choisi dans le groupe constitué par -S-, -CH₂-, -SCH₂-, -CH₂S-, -CH(OH)-, -C(O)-, -CX^{a}₂-, -CX^{a}H-, -CH₂O- et -OCH₂-, et X^{a} est halogène.

5. Utilisation selon la revendication 1, dans laquelle Y est -O- ou -CH₂-.

6. Utilisation selon la revendication 1, dans laquelle Ar est phényle ou pyridinyle.

7. Utilisation selon la revendication 4, dans laquelle B est phényle et Ar est phényle ou pyridinyle.

8. Utilisation selon la revendication 5, dans laquelle B est phényle et Ar est phényle ou pyridinyle.

9. Utilisation selon la revendication 6, dans laquelle B est phényle.

10. Utilisation selon la revendication 1, dans laquelle le composé est l'une des formules ou un de leurs sels pharmaceutiquement acceptables: 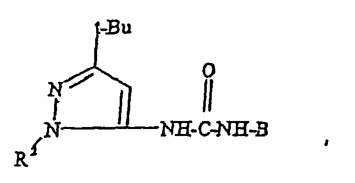 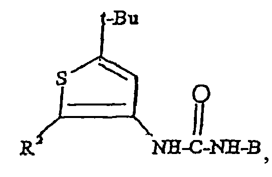 ou 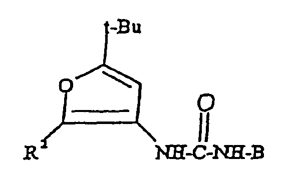 où B et R² sont tels que définis dans la revendication 1.

11. Utilisation selon la revendication 3, dans laquelle R² est choisi parmi des membres substitués ou non substitués du groupe constitué par phényle et pyridinyle, et où, si R² est un groupe substitué, il est substitué par un ou plusieurs substituants choisis dans le groupe constitué par halogène et Wₙ, où n est 0-3, et W est choisi dans le groupe constitué par -NO₂, alkyle en C₁-C₃, -NH(O)CH₃, -CF₃, -OCH₃, -F, -Cl, -NH₂, -SO₂CH₃, pyridinyle, phényle, phényle jusqu'à perhalogéné et phényle substitué par alkyle en C₁-C₆.

12. Utilisation selon la revendication 1, dans laquelle le composé de formule I présente une activité sur le p38 (CI₅₀) meilleure que 10 µM, selon une détermination par un essai de kinase *in vitro.*

13. Utilisation selon la revendication 1, dans laquelle la maladie est la polyarthrite rhumatoïde, l'arthrose, l'arthrite septique, l'ostéoporose, l'ostéoporose post-ménopausique, l'asthme, le choc septique, une maladie inflammatoire chronique de l'intestin, ou le résultat de réactions de rejet de greffes.
